# EUROPÄISCHE PATENTANMELDUNG

(11) **EP 2 123 621 A1**
(43) Veröffentlichungstag der Anmeldung: **25.11.2009**
(21) Anmeldenummer: 08075510.1
(22) Anmeldetag: 20.05.2008
(51) Int. Cl.: C07B 59/00, C07C 229/24, C07C 237/06, A61K 51/04, A61K 31/04, A61K 31/195

(54) **Neue {F-18}-markierte L-Glutaminsäure- und L-Glutaminderivate (I), ihre Verwendung sowie Verfahren zu ihrer Herstellung**

(71) Anmelder: Bayer Schering Pharma Aktiengesellschaft, 13353 Berlin (DE)
(72) Erfinder: Friebe, Dr. Matthias, 13509 Berlin (DE); Schmitt-Willich, Dr. Heribert, 10551 Berlin (DE); Berndt, Dr. Matthias, 12163 Berlin (DE); Koglin, Dr. Norman, 12587 Berlin (DE); Sülze, Dr. Detlev, 13467 Berlin (DE); Dinkelborg, Dr. Ludger, 13465 Berlin (DE)

(57) **Zusammenfassung**

Es werden die Verbindungen und die Synthese von [F-18] markierter L-Glutaminsäure, [F-18] markiertem L-Glutamat, ihren Derivaten gemäß Formel (I) und ihre Verwendungen beschrieben.

## Beschreibung

Die Erfindung betrifft die in den Patentansprüchen gekennzeichneten Gegenstände, nämlich [F-18] markierte L-Glutaminsäurederivate und [F-18] markierte L-Glutaminderivate der allgemeinen Formel I, sowie ihre Verwendung und Verfahren zu ihrer Herstellung.

Die frühe Diagnose von malignen Tumorerkrankungen spielt eine sehr wichtige Rolle für die Überlebensprognose eines Tumorpatienten. Bei dieser Diagnose sind nicht-invasive, bildgebende diagnostische Verfahren ein wichtiges Hilfsmittel. In den letzten Jahren hat sich hier vor allem die PET-Technologie (Positronen-Emissions-Tomographie) als besonders nützlich erwiesen. Die Sensitivität und Spezifität der PET-Technologie hängt wesentlich von der verwendeten signalgebenden Substanz (Tracer) und ihrer Verteilung im Körper ab. Bei der Suche nach geeigneten Tracern versucht man bestimmte Eigenschaften von Tumoren auszunutzen, die Tumorgewebe von gesundem, umliegenden Gewebe unterscheiden. Das bevorzugte kommerziell genutzte Isotop, welches für PET Anwendung findet, ist ¹⁸F. ¹⁸F stellt durch seine kurze Halbwertszeit von unter 2 Stunden besondere Anforderungen an die Herstellung geeigneter Tracer. Aufwändige, lange Synthesewege und Aufreinigungen sind mit diesem Isotop nicht möglich, da sonst ein erheblicher Teil der Radioaktivität des Isotops bereits abgeklungen ist, bevor der Tracer zur Diagnose eingesetzt werden kann. Es ist deshalb häufig nicht möglich etablierte Synthesewege für nichtradioaktive Fluorierungen auf die Synthese von ¹⁸F-Tracern anzuwenden. Des weiteren führt die hohe spezifische Aktivität des ¹⁸F (ca. 80 GBq/nmol) zu sehr niedrigen Substanzmengen an [¹⁸F]Fluorid für die Tracersynthese, was wiederum einen extremen Überschuß an Präkursor bedingt und den Erfolg einer auf nicht-radioaktiven Fluorierungsreaktionen basierender Radiosynthesestrategie unvorhersehbar gestaltet.

FDG ([¹⁸F]2-Fluorodesoxyglukose)-PET ist ein weithin akzeptiertes und verbreitetes Hilfsmittel in der Diagnose und weiteren klinischen Verfolgung von Tumorerkrankungen. Maligne Tumore konkurrieren mit dem Wirtsorganismus um Glukoseversorgung zur Nährstoffversorgung (Warburg O. Über den Stoffwechsel der Carcinomzelle. Biochem. Zeitschrift 1924; 152: 309-339; Kellof G. Progress and Promise of FDG-PET Imaging for Cancer Patient Management and Oncologic Drug Development. Clin Cancer Res. 2005; 11 (8): 2785-2807) Dabei haben Tumorzellen im Vergleich zu umliegenden Zellen des Normalgewebes gewöhnlich einen erhöhten Glukosestoffwechsel. Dies wird bei der Verwendung von Fluorodesoxyglukose (FDG), einem Glukosederivat genutzt, welches verstärkt in die Zellen transportiert wird, dort aber nach der Phosphorylierung als FDG-6-phosphat metabolisch eingeschlossen wird ("Warburg-Effekt"). ¹⁸F markiertes FDG ist daher ein wirkungsvoller Tracer zur Detektion von Tumorerkrankungen beim Patienten mittels der PET-Technologie. Auf der Suche nach neuen PET Tracern wurden in jüngster Zeit auch zunehmend Aminosäuren für die ¹⁸F PET Bildgebung eingesetzt (z. B. (review): Eur J Nucl Med Mol Imaging. 2002 May;29(5):681-90). Dabei eignen sich einige der ¹⁸F markierten Aminosäuren für die Messung der Geschwindigkeitsrate der Proteinsynthese, die meisten anderen Derivate aber für die Messung der direkten Zellaufnahme im Tumor. Bekannte ¹⁸F markierte Aminosäuren sind z. B. von Tyrosin-, Phenylalanin-, Prolin-, Asparagin- und unnatürlichen Aminosäuren abgeleitet (z. B. J. Nucl Med 1991; 32:1338-1346, J Nucl Med 1996; 37:320-325, J Nucl Med 2001;42:752-754 bzw. J Nucl Med 1999; 40:331-338.). Glutaminsäure und Glutamin sind als ¹⁸F markierte Derivate nicht bekannt, wohingegen nicht-radioaktive fluorierte Glutamin- und Glutaminsäurederivate geläufig sind; so z. Bsp. jene, die an γ-Position (z. Bsp. (review): Amino Acids. (2003) Apr;24(3):245-61) oder an β-Position (z. Bsp. Tetrahedron Lett.; 30; 14; 1989; 1799-1802, J. Org. Chem.; 54; 2; 1989; 498-500, Tetrahedron: Asymmetry; 12; 9; 2001; 1303 - 1312) Fluor aufweisen.
Von Glutaminsäurederivaten, die an den chemischen Funktionalitäten Schutzgruppen und in β oder γ Position eine Abgangsgruppe aufweisen, ist in der Vergangenheit bereits berichtet worden. So wurde über Glutamat als Mesylat bzw. Bromid in γ-Position informiert, deren Säure- und Aminfunktionen mit Ester- bzw. Z-Schutzgruppen versehen waren (J. Chem. Soc. Perkin Trans. 1; 1986; 1323-1328) oder beispielsweise von γ-Chloro-Glutaminsäure ohne Schutzgruppen (Synthesis; (1973); 44-46). Über ähnliche Derivate, bei denen aber die Fluchtgruppe in β-Stellung positioniert ist, wurde ebenfalls verschiedentlich berichtet: z. Bsp. Chem. Pharm. Bull.; 17; 5; (1969); 879-885, J.Gen.Chem.USSR (Engl.Transl.); 38; (1968); 1645-1648; Tetrahedron Lett.; 27; 19; (1986); 2143-2144, Chem. Pharm. Bull.; EN; 17; 5; 1969; 873-878, Patent FR 1461184, Patent JP 13142.)

Die derzeitigen PET-Tracer, die für die Tumordiagnostik eingesetzt werden, haben einige unbestrittene Nachteile: So reichert sich FDG zwar bevorzugt in solchen Zellen mit erhöhtem Glukosestoffwechsel an, es gibt allerdings auch in anderen pathologischen und physiologischen Zuständen einen erhöhten Glukosestoffwechsel in den beteiligten Zellen und Geweben, z. Bsp. Infektionsherde oder Wundheilung (zusammengefasst in J. Nucl. Med. Technol. (2005), 33, 145-155). Es ist häufig immer noch schwierig zu entscheiden, ob eine mittels FDG-PET detektierte Läsion tatsächlich neoplastischen Ursprungs ist oder auf andere physiologische oder pathologische Zustände des Gewebes zurückzuführen ist. Insgesamt weist die Diagnosetätigkeit mittels FDG-PET in der Onkologie eine Sensitivität von 84% und eine Spezifität von 88% auf (Gambhir et al. "A tabulated summary of the FDG PET literature" J. Nucl. Med. 2001, 42, 1-93S). Tumore im Gehirn lassen sich beispielsweise durch die hohe Anreicherung von FDG in gesundem Hirngewebe nur sehr schwer darstellen.
Die bisher bekannten ¹⁸F markierten Aminosäurederivate sind in einigen Fällen gut geeignet, um Tumore im Gehirn zu detektieren ((review): Eur J Nucl Med Mol Imaging. 2002 May;29(5):681-90), allerdings können sie bei anderen Tumoren nicht mit den Bildgebungseigenschaften des "Goldstandards" [¹⁸F]2-FDG konkurrieren. Die metabolische Anreicherung und Retention der bislang F-18 markierten Aminosäuren in tumorösem Gewebe ist in der Regel niedriger als für FDG. Darüberhinaus, ist die Zugänglichkeit isomerenreiner F-18 markierter nichtaromatischer Aminosäuren chemisch höchst anspruchsvoll.
Ähnlich wie für Glukose wurde auch für Glutaminsäure und Glutamin ein erhöhter Metabolismus in proliferierenden Tumorzellen beschrieben (Medina, J Nutr. 1131:2539S-2542S, 2001; Souba, Ann Surg 218: 715-728, 1993). Die erhöhte Rate an Protein- und Nukleinsäuresynthesen sowie die Energiegewinnung per se werden als Gründe für einen verstärkten Glutaminkonsum von Tumorzellen angenommen. Die Synthese von entsprechenden C-11 und C-14 markierten, mit dem natürlichen Substrat also identischen Verbindungen, wurde in der Literatur bereits beschrieben (z. Bsp. Antoni, Enzyme Catalyzed Synthesis of L-[4-C-11]Aspartate and L-[5-C-11]Glutamate. J. Labelled Compd. Radiopharm. 44;( 4) 2001: 287 - 294) und Buchanan, The biosynthesis of showdomycin: studies with stable isotopes and the determination of principal precursors. J. Chem. Soc. Chem. Commun.; EN; 22; 1984; 1515-1517). Erste Hinweise mit der C-11 markierten Verbindung deuten auf keine signifikante Tumorakkumulation hin.

Aufgabe der vorliegenden Erfindung ist es neue Verbindungen zu finden, die sich in [¹⁸F] markierter Form für die PET-basierte Diagnose eignen.

Die Aufgabe wird gelöst durch die erfindungsgemäße Bereitstellung von [¹⁸F] markierten Glutaminsäurederivaten und [¹⁸F] markierten Glutaminderivaten, gemäß der allgemeinen Formel (I), einschließlich ihrer Diastereomere und Enantiomere: 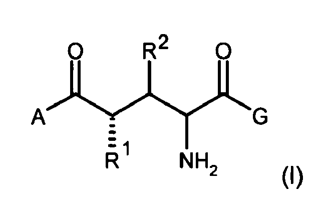 worin
A für
a) Hydroxyl,
b) verzweigtes oder unverzweigtes C₁-C₅ alkoxy,
c) verzweigtes oder unverzweigtes Hydroxy C₁-C₅ Alkoxy,
d) verzweigtes oder unverzweigtes O-C₁-C₅ Alkyl-(O-C₁-C₄ alkyl)ₙ-O-C₁-C₄ alkyl,
e) N(C₁-C₅ Alkyl)₂,
f) NH₂,
g) N(H)-L,
h) O-L oder
i) O-Z
   steht,
G für
a) Hydroxyl,
b) O-Z,
b) verzweigtes oder unverzweigtes O-C₁-C₅ Alkyl,
c) verzweigtes oder unverzweigtes O-C₂-C₅ Alkenyl,
d) verzweigtes oder unverzweigtes O-C₁-C₅ Alkyl-(O-C₁-C₄ alkyl)ₙ-O-C₁-C₄ alkyl,
e) verzweigtes oder unverzweigtes O-C₂-C₅ Alkinyl, oder
f) Triphenylmethoxy
   steht,
R¹ und R² für
a) Wasserstoff,
b) verzweigtes oder unverzweigtes ¹⁸F-C₁-C₅ Alkoxy,
c) verzweigtes oder unverzweigtes ¹⁸F-C₁-C₅ Alkyl,
d) verzweigtes oder unverzweigtes ¹⁸F-C₂-C₅ Alkenyl,
e) verzweigtes oder unverzweigtes ¹⁸F-C₂-C₅ Alkinyl,
f) Hydroxyl,
g) verzweigtes oder unverzweigtes C₁-C₅ Alkyl oder
h) verzweigtes oder unverzweigtes C₁-C₅ Alkoxy
   steht,
   mit der Maßgabe, dass einer der Substituenten R¹ oder R² genau ein ¹⁸F Isotop beinhaltet und der jeweils andere Substituent kein ¹⁸F Isotop beinhaltet, mit der Maßgabe dass R¹ nicht Wasserstoff ist,
L für
a) verzweigtes oder unverzweigtes C₁-C₅ Alkyl,
b) verzweigtes oder unverzweigtes C₂-C₅ Alkenyl,
c) verzweigtes oder unverzweigtes C₁-C₅ Alkyl-(O-C₁-C₄ alkyl)ₙ-O-C₁-C₄ alkyl oder
d) verzweigtes oder unverzweigtes C₂-C₅ Alkinyl,
   steht,
Z für ein Metallkationenequivalent steht, und
wobei
n = 0, 1, 2 oder 3.

Bevorzugte Verbindungen der Erfindung gemäß der Formel (I) zeichnen sich dadurch aus, dass
A für
a) Hydroxyl,
b) Methoxy,
c) Ethoxy,
d) Propoxy,
e) NMe₂,
f) NEt_{2 ,}
g) NH₂,
h) N(H)-L oder
i) O-L,
j) O-Z
steht.

Weitere bevorzugte Verbindungen der Erfindung gemäß der Formel (I) zeichnen sich dadurch aus, dass
A für
a) Hydroxyl,
b) Methoxy,
c) Ethoxy,
d) NMe₂,
e) NH₂, oder
f) N(H)-L
steht.

Besonders bevorzugte Verbindungen der Erfindung gemäß der Formel (I) zeichnen sich dadurch aus, dass
A für
a) Hydroxyl,
b) verzweigtes oder unverzweigtes C₁-C₅ Alkoxy oder
c) NH₂
steht.

Bevorzugte Verbindungen der Erfindung gemäß der Formel (I) zeichnen sich dadurch aus, dass
A für
Hydroxyl
steht.

Bevorzugte Verbindungen der Erfindung gemäß der Formel (I) zeichnen sich dadurch aus, dass
A für
NH₂
steht.

Bevorzugte Verbindungen der Erfindung gemäß der Formel (I) zeichnen sich dadurch aus, dass
A für
Ethoxy
steht.

Bevorzugte Verbindungen der Erfindung gemäß der Formel (I) zeichnen sich dadurch aus, dass
G für
a) Hydroxyl,
b) verzweigtes oder unverzweigtes O-C₁-C₄ Alkyl, oder
c) O-C₂H₄-OMe
steht.

Weitere bevorzugte Verbindungen der Erfindung gemäß der Formel (I) zeichnen sich dadurch aus, dass
G für
a) Hydroxyl, oder
b) verzweigtes oder unverzweigtes O-C₁-C₄ Alkyl
steht.

Besonders bevorzugte Verbindungen der Erfindung gemäß der Formel (I) zeichnen sich dadurch aus, dass
G für
a) Hydroxyl,
b) Methoxy oder
c) Ethoxy
steht.

Besonders bevorzugte Verbindungen der Erfindung gemäß der Formel (I) zeichnen sich dadurch aus, dass
G für
Methoxy
steht.

Bevorzugte Verbindungen der Erfindung gemäß der Formel (I) zeichnen sich dadurch aus, dass
R¹ und R² für
a) Wasserstoff,
b) verzweigtes oder unverzweigtes ¹⁸F-C₂-C₄ Alkoxy,
c) verzweigtes oder unverzweigtes ¹⁸F-C₃-C₅ Alkyl,
d) verzweigtes oder unverzweigtes ¹⁸F-C₃-C₅ Alkenyl,
e) verzweigtes oder unverzweigtes ¹⁸F-C₃-C₅ Alkinyl,
f) Hydroxyl,
g) verzweigtes oder unverzweigtes C₁-C₅ Alkyl oder
h) verzweigtes oder unverzweigtes C₁-C₅ Alkoxy
   steht,
   mit der Maßgabe, dass einer der Substituenten R¹ oder R² genau ein ¹⁸F Isotop beinhaltet und der jeweils andere Substituent kein ¹⁸F Isotop beinhaltet, mit der Maßgabe dass R¹ nicht Wasserstoff ist,

Ein weiterer besonderer Gegenstand der Erfindung sind Verbindungen der allgemeinen Formel I worin
R¹ für
a) verzweigtes oder unverzweigtes ¹⁸F-C₂ Alkoxy,
b) verzweigtes oder unverzweigtes ¹⁸F-C₃ Alkyl,
c) verzweigtes oder unverzweigtes ¹⁸F-C₃ Alkenyl, oder
d) verzweigtes oder unverzweigtes ¹⁸F-C₃ Alkinyl
steht.

Unverzweigtes ¹⁸F-C₂ Alkoxy ist ¹⁸F-Ethoxy.
Unverzweigtes ¹⁸F-C₃ Alkyl ist ¹⁸F-Propyl.
Unverzweigtes ¹⁸F-C₃ Alkenyl ist ¹⁸F-Propenyl.
Unverzweigtes ¹⁸F-C₃ Alkinyl ist ¹⁸F-Propinyl.

Ein weiterer besonderer Gegenstand der Erfindung sind Verbindungen der allgemeinen Formel I worin
R¹ für ¹⁸F-Ethoxy oder ¹⁸F-Propyl und R² für Wasserstoff steht.

Weitere bevorzugte Verbindungen der Erfindung gemäß der Formel (I) zeichnen sich dadurch aus, dass
R¹ ausgewählt ist aus der Gruppe, ¹⁸F-ethoxy, ¹⁸F-propoxy, ¹⁸F-methyl, ¹⁸F-ethyl und ¹⁸F-propyl und R² Wasserstoff ist.

Weitere bevorzugte Verbindungen der Erfindung gemäß der Formel (I) zeichnen sich dadurch aus, dass R¹ für ¹⁸F und R² für Wasserstoff steht.

Bevorzugte Verbindungen der Erfindung gemäß der Formel (I) zeichnen sich dadurch aus, dass
L für
a) Methyl,
b) Ethyl,
c) Propyl,
d) iso-Propyl,
e) -C₂H₄-OMe, oder
f) -C₂H₄-O-C₂H₄-OMe
steht.

Besonders bevorzugte Verbindungen der Erfindung gemäß der Formel (I) zeichnen sich dadurch aus, dass
L für
a) Methyl oder
b) Ethyl
steht.

Ebenfalls bevorzugte Verbindungen der Erfindung gemäß der Formel (I) zeichnen sich dadurch aus, dass Z ist ausgewählt aus der Gruppe Na⁺, K⁺, Ca²⁺ und Mg²⁺.
Bevorzugtes Z ist Na⁺.

Das Verfahren zur Herstellung der erfindungsgemäßen Verbindungen der allgemeinen Formel (I) zeichnet sich dadurch aus indem
- eine oder mehrere vorhandene Schutzgruppen einer Verbindung nach Formel (II) abgespalten wird oder werden.

Verbindungen nach Formel I: 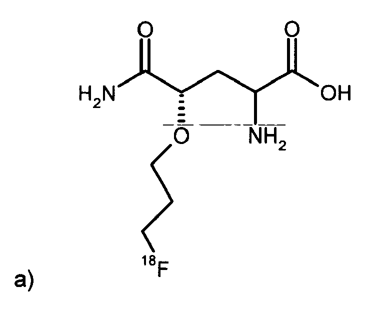 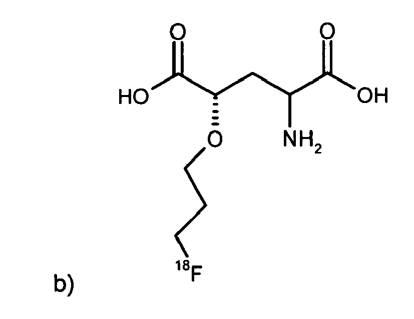 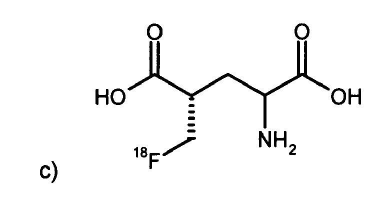 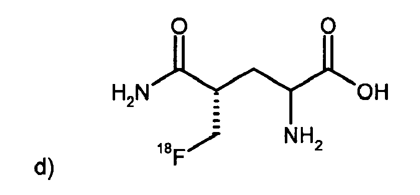 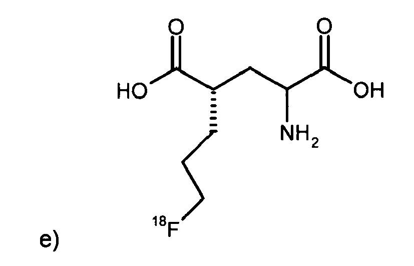 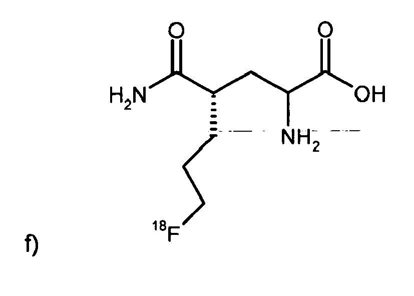 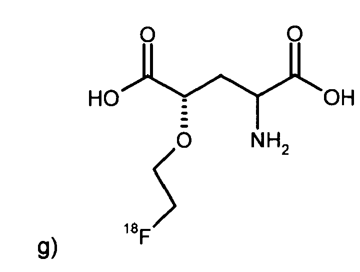 und

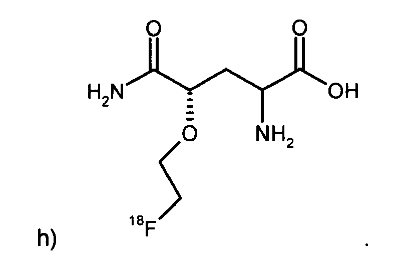

In einem weiteren Aspekt bezieht sich somit die vorliegende Erfindung auf Verbindungen der allgemeinen Formel (II): 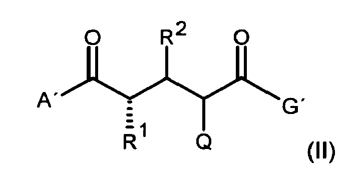 worin,
A' für
a) Hydroxyl,
b) verzweigtes oder unverzweigtes C₁-C₅ alkoxy,
c) verzweigtes oder unverzweigtes Hydroxy C₁-C₅ Alkoxy,
d) verzweigtes oder unverzweigtes O-C₁-C₅ Alkyl-(O-C₁-C₄ alkyl)ₙ-O-C₁-C₄ alkyl,
e) N(C₁-C₅ Alkyl)₂,
f) NH₂,
g) N(H)-L', oder
h) O-L'
   steht,
G' für
a) hydroxyl,
b) O-Z',
c) verzweigtes oder unverzweigtes O-C₁-C₅ Alkyl,
d) verzweigtes oder unverzweigtes O-C₂-C₅ Alkenyl,
e) verzweigtes oder unverzweigtes O-C₁-C₅ Alkyl-(O-C₁-C₄ alkyl)ₙ-O-C₁-C₄ alkyl,
f) verzweigtes oder unverzweigtes O-C₂-C₅ Alkinyl, oder
g) Triphenylmethoxy
   steht,
R¹ und R² für
a) Wasserstoff,
b) verzweigtes oder unverzweigtes ¹⁸F C₂-C₅ Alkoxy,
c) verzweigtes oder unverzweigtes ¹⁸F-C₁-C₅ Alkyl,
d) verzweigtes oder unverzweigtes ¹⁸F-C₂-C₅ Alkenyl,
e) verzweigtes oder unverzweigtes ¹⁸F-C₂-C₅ Alkinyl,
f) Hydroxyl,
g) verzweigtes oder unverzweigtes C₁-C₅ Alkyl, oder
h) verzweigtes oder unverzweigtes C₁-C₅ Alkoxy
   steht,
mit der Maßgabe, dass genau einer der Substituenten R¹ oder R² genau ein ¹⁸F Isotop und der jeweils andere Substituent kein ¹⁸F Isotop beinhaltet, mit der Maßgabe dass R¹ nicht Wasserstoff ist,
Q für
a) N(H)-tert-Butoxycarbonyl,
b) N(H)-Allyloxycarbonyl,
c) N(H)-Benzyloxycarbonyl,
d) N(H)-Ethoxycarbonyl,
e) N(H)-Methoxycarbonyl,
f) N(H)-Propoxycarbonyl,
e) N(H)-2,2,2-Trichlorethoxycarbonyl,
f) N(H)-1,1-Dimethylpropinyl,
g) N(H)-1-Methyl-1-phenyl-ethoxycarbonyl,
h) N(H)-1-Methyl-1-(4-biphenylyl)-ethoxycarbonyl,
i) N(H)-cyclobutylcarbonyl,
j) N(H)-1-Methylcyclobutylcarbonyl,
k) N(H)-Vinylcarbonyl,
l) N(H)-Allylcarbonyl,
m) N(H)-Adamantylcarbonyl,
n) N(H)-Diphenylmethylcarbonyl,
o) N(H)-Cinnamylcarbonyl,
p) N(H)-Formyl,
q) N(H)-Benzoyl,
r) N(H)-Trityl,
s) N(H)-p-Methoxyphenyl-diphenylmethyl,
t) N(H)-di-(p-Methoxyphenyl)-phenylmethyl,
u) 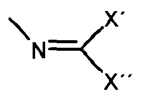 oder
v) N-(tert-Butoxycarbonyl)₂,
   steht,
L' für
a) verzweigtes oder unverzweigtes C₁-C₅ Alkyl,
b) verzweigtes oder unverzweigtes C₂-C₅ Alkenyl,
c) verzweigtes oder unverzweigtes C₁-C₅ Alkyl-(O-C₁-C₄ alkyl)ₙ-O-C₁-C₄ alkyl, oder
d) verzweigtes oder unverzweigtes C₂-C₅ Alkinyl
   steht,
X' und X'' unabhängig voneinander für
a) verzweigtes oder unverzweigtes C₁-C₅ Alkyl,
b) substituiertes oder unsubstituiertes Aryl,
c) substituiertes oder unsubstituiertes Aralkyl oder
d) substituiertes oder unsubstituiertes Heteroaryl
   steht,
Z' für ein Metallkationenequivalent
steht, und
wobei n = 0, 1, 2 oder 3 ist.

Bevorzugte Verbindungen der Erfindung gemäß der Formel (II) zeichnen sich dadurch aus, dass
A' für
a) Hydroxyl,
b) Methoxy,
c) Ethoxy,
d) Propoxy,
e) NMe₂,
f) NEt_{2 ,}
g) NH₂,
h) N(H)-L oder
i) O-L,
j) O-Z
steht.

Weitere bevorzugte Verbindungen der Erfindung gemäß der Formel (II) zeichnen sich dadurch aus, dass
A' für
a) Hydroxyl,
b) Methoxy,
c) Ethoxy,
d) NMe₂,
e) NH₂, oder
f) N(H)-L
steht.

Besonders bevorzugte Verbindungen der Erfindung gemäß der Formel (II) zeichnen sich dadurch aus, dass
A' für
a) Hydroxyl,
b) verzweigtes oder unverzweigtes C₁-C₅ Alkoxy oder
c) NH₂
steht.

Bevorzugte Verbindungen der Erfindung gemäß der Formel (II) zeichnen sich dadurch aus, dass
A' für
Hydroxyl
steht.

Bevorzugte Verbindungen der Erfindung gemäß der Formel (II) zeichnen sich dadurch aus, dass
A'für
NH₂
steht.

Bevorzugte Verbindungen der Erfindung gemäß der Formel (II) zeichnen sich dadurch aus, dass
A' für
Ethoxy
steht.

Bevorzugte Verbindungen der Erfindung gemäß der Formel (II) zeichnen sich dadurch aus, dass
G'für
a) Hydroxyl,
b) verzweigtes oder unverzweigtes O-C₁-C₄ Alkyl, oder
c) O-C₂H₄-OMe
steht.

Weitere bevorzugte Verbindungen der Erfindung gemäß der Formel (II) zeichnen sich dadurch aus, dass
G' für
a) Hydroxyl, oder
b) verzweigtes oder unverzweigtes O-C₁-C₄ Alkyl
steht.

Besonders bevorzugte Verbindungen der Erfindung gemäß der Formel (II) zeichnen sich dadurch aus, dass
G' für
a) Hydroxyl,
b) Methoxy oder
c) Ethoxy
steht.

Besonders bevorzugte Verbindungen der Erfindung gemäß der Formel (II) zeichnen sich dadurch aus, dass
G' für
Methoxy
steht.

Bevorzugte Verbindungen der Erfindung gemäß der Formel (II) zeichnen sich dadurch aus, dass
R¹ und R² für
a) Wasserstoff,
b) verzweigtes oder unverzweigtes ¹⁸F-C₂-C₄ Alkoxy,
c) verzweigtes oder unverzweigtes ¹⁸F-C₃-C₅ Alkyl,
d) verzweigtes oder unverzweigtes ¹⁸F-C₃-C₅ Alkenyl, oder
e) verzweigtes oder unverzweigtes ¹⁸F-C₃-C₅ Alkinyl,
f) Hydroxyl,
g) verzweigtes oder unverzweigtes C₁-C₅ Alkyl oder
h) verzweigtes oder unverzweigtes C₁-C₅ Alkoxy
steht,
mit der Maßgabe, dass genau einer der Substituenten R¹ oder R² genau ein ¹⁸F-Isotop beinhaltet und der jeweils andere Substituent Wasserstoff ist, mit der Maßgabe dass R¹ nicht Wasserstoff ist.

Bevorzugte Verbindungen der Erfindung gemäß der Formel (II) zeichnen sich dadurch aus, dass
R¹ ausgewählt ist aus der Gruppe ¹⁸F-ethoxy, ¹⁸F-propoxy, ¹⁸F-methyl, ¹⁸F-ethyl und ¹⁸F-propyl und R² Wasserstoff ist.

Ein weiterer besonderer Gegenstand der Erfindung sind Verbindungen der allgemeinen Formel I worin
R¹ für
e) verzweigtes oder unverzweigtes ¹⁸F-C₂ Alkoxy,
f) verzweigtes oder unverzweigtes ¹⁸F-C₃ Alkyl,
g) verzweigtes oder unverzweigtes ¹⁸F-C₃ Alkenyl, oder
h) verzweigtes oder unverzweigtes ¹⁸F-C₃ Alkinyl
steht.

Unverzweigtes ¹⁸F-C₂ Alkoxy ist ¹⁸F-Ethoxy.
Unverzweigtes ¹⁸F-C₃ Alkyl ist ¹⁸F-Propyl.
Unverzweigtes ¹⁸F-C₃ Alkenyl ist ¹⁸F-Propenyl.
Unverzweigtes ¹⁸F-C₃ Alkinyl ist ¹⁸F-Propinyl.

Ein weiterer besonderer Gegenstand der Erfindung sind Verbindungen der allgemeinen Formel I worin
R¹ für ¹⁸F-Ethoxy oder ¹⁸F-Propyl und R² für Wasserstoff steht.

Bevorzugte Verbindungen der Erfindung gemäß der Formel (II) zeichnen sich dadurch aus, dass
L' für
a) Methyl,
b) Ethyl,
c) Propyl,
d) iso-Propyl,
e) -C₂H₄-OMe, oder
f) -C₂H₄-O-C₂H₄-OMe
steht.

Besonders bevorzugte Verbindungen der Erfindung gemäß der Formel (II) zeichnen sich dadurch aus, dass
L' für
a) Methyl oder
b) Ethyl
steht.

Ebenfalls bevorzugte Verbindungen der Erfindung gemäß der Formel (II) zeichnen sich dadurch aus, dass Z' ist ausgewählt aus der Gruppe Na⁺, K⁺, Ca²⁺ und Mg²⁺.
Bevorzugtes Z' ist Na⁺.

Bevorzugte Verbindungen der Erfindung gemäß der Formel (II) zeichnen sich dadurch aus, dass
Q für
a) N(H)-tert-Butoxycarbonyl,
b) N(H)-Benzyloxycarbonyl,
c) N-(tert-Butoxycarbonyl)₂, oder
d) 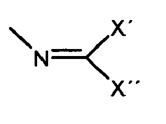 steht.

Weitere bevorzugte Verbindungen der Erfindung gemäß der Formel (II) zeichnen sich dadurch aus, dass
Q für
a) N(H)-tert-Butoxycarbonyl,
b) N-(tert-Butoxycarbonyl)₂, oder
c) 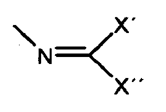
steht.

Besonders bevorzugte Verbindungen der Erfindung gemäß der Formel (II) zeichnen sich dadurch aus, dass
Q für
a) N(H)-tert-Butoxycarbonyl, oder
b) 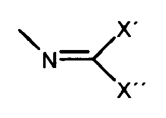
steht.

Besonders bevorzugte Verbindungen der Erfindung gemäß der Formel (II) zeichnen sich dadurch aus, dass
Q für N(H)-tert-Butoxycarbonyl steht.

Bevorzugte Verbindungen der Erfindung gemäß der Formel (II) zeichnen sich dadurch aus, dass
L' für
a) Methyl,
b) Ethyl,
c) Propyl,
d) iso-Propyl,
e) -C₂H₄-OMe, oder
f) -C₂H₄-O-C₂H₄-OMe
steht.

Bevorzugte Verbindungen der Erfindung gemäß der Formel (II) zeichnen sich dadurch aus, dass
X' und X'' unabhängig voneinander für
a) verzweigtes oder unverzweigtes C₁-C₅ Alkyl,
b) substituiertes oder unsubstituiertes Aryl oder
c) substituiertes oder unsubstituiertes Aralkyl
steht.

Weitere bevorzugte Verbindungen der Erfindung gemäß der Formel (II) zeichnen sich dadurch aus, dass
X' und X'' unabhängig voneinander für
a) verzweigtes oder unverzweigtes C₁-C₅ Alkyl, oder
b) substituiertes oder unsubstituiertes Aryl
steht.

Besonders bevorzugte Verbindungen der Erfindung gemäß der Formel (II) zeichnen sich dadurch aus, dass
X' und X'' für Phenyl bzw. für in 2-Position substituiertes Phenyl
steht.

Das Verfahren zur Herstellung der erfindungsgemäßen Verbindungen der allgemeinen Formel (II) zeichnet sich dadurch aus, dass die Mehrzahl der Verbindung nach Formel (II), aus einer Verbindung der Formel (III) nach Einführung des ¹⁸F Isotops entstehen kann.

Verfahren zur Herstellung von Verbindungen der allgemeinen Formel (II) indem
- eine Verbindung nach Formel (III) mit F-18 Fluorid umgesetzt wird.

Verbindungen nach Formel I, II oder III zur Verwendung als Arzneimittel.

Verbindungen nach Formel I, II oder III zur Verwendung für Bildgebung bei Tumorerkrankungen.

Verwendung von Verbindungen nach Formel I, II oder III zur Herstellung eines Arzneimittels für Bildgebung bei Tumorerkrankungen.

In einem weiteren Aspekt bezieht sich somit die vorliegende Erfindung auf Verbindungen der allgemeinen Formel (III): 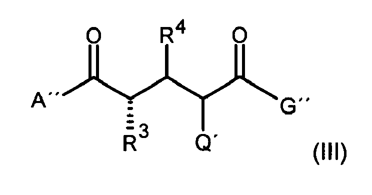 worin
A" für
a) verzweigtes oder unverzweigtes C₁-C₅ alkoxy,
b) verzweigtes oder unverzweigtes Hydroxy C₁-C₅ Alkoxy,
c) verzweigtes oder unverzweigtes O-C₁-C₅ Alkyl-(O-C₁-C₄ alkyl)ₙ-O-C₁-C₄ alkyl,
d) N(C₁-C₅ Alkyl)₂,
e) NH₂,
f) N(H)-L" oder
g) O-L''
steht,
G" für
a) O-Z'',
b) verzweigtes oder unverzweigtes O-C₁-C₅ Alkyl,
c) verzweigtes oder unverzweigtes O-C₂-C₅ Alkenyl,
d) verzweigtes oder unverzweigtes O-C₁-C₅ Alkyl-(O-C₁-C₄ alkyl)ₙ-O-C₁-C₄ alkyl,
e) verzweigtes oder unverzweigtes O-C₂-C₅ Alkinyl, oder
f) Triphenylmethoxy
steht,
R³ und R⁴ für
a) Wasserstoff,
b) verzweigtes oder unverzweigtes E-C₂-C₅ Alkoxy,
c) verzweigtes oder unverzweigtes E-C₁-C₅ Alkyl,
d) verzweigtes oder unverzweigtes E-C₂-C₅ Alkenyl,
e) verzweigtes oder unverzweigtes E-C₂-C₅ Alkinyl,
f) Hydroxyl,
g) verzweigtes oder unverzweigtes C₁-C₅ Alkyl, oder
h) verzweigtes oder unverzweigtes C₁-C₅ Alkoxy
steht, mit der Maßgabe, dass genau einer der Substituenten R³ oder R⁴ ein E beinhaltet und der jeweils andere Substituent kein E beinhaltet, mit der Maßgabe dass R³ nicht Wasserstoff ist,
E für
a) Chloro,
b) Bromo,
c) Methansulfonyloxy,
d) Trifluormethansulfonyloxy,
e) Nonafluorbutyloxy,
f) Tosyloxy oder
g) Iodo
steht,
Q' für
a) N(H)-tert-Butoxycarbonyl,
b) N(H)-Allyloxycarbonyl,
c) N(H)-Benzyloxycarbonyl,
d) N(H)-Ethoxycarbonyl,
e) N(H)-Methoxycarbonyl,
f) N(H)-Propoxycarbonyl,
g) N(H)-2,2,2-Trichlorethoxycarbonyl,
h) N(H)-1,1-Dimethylpropinyl,
i) N(H)-1-Methyl-1-phenyl-ethoxycarbonyl,
j) N(H)-1-Methyl-1-(4-biphenylyl)-ethoxycarbonyl,
k) N(H)-Cyclobutylcarbonyl,
l) N(H)-1-Methylcyclobutylcarbonyl,
m) N(H)-Vinylcarbonyl,
n) N(H)-Allylcarbonyl,
o) N(H)-Adamantylcarbonyl,
p) N(H)-Diphenylmethylcarbonyl,
q) N(H)-Cinnamylcarbonyl,
r) N(H)-Formyl,
s) N(H)-Benzoyl,
t) N(H)-Trityl,
u) N(H)-p-Methoxyphenyl-diphenylmethyl,
v) N(H)-di-(p-Methoxyphenyl)-phenylmethyl,
w) 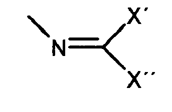 oder
x) N-(tert-Butoxycarbonyl)₂,
steht,
L'' für
a) verzweigtes oder unverzweigtes C₁-C₅ Alkyl,
b) verzweigtes oder unverzweigtes C₂-C₅ Alkenyl,
c) verzweigtes oder unverzweigtes C₁-C₅ Alkyl-(O-C₁-C₄ alkyl)ₙ-O-C₁-C₄ alkyl oder
d) verzweigtes oder unverzweigtes C₂-C₅ Alkinyl
steht,
X' and X'' unabhängig voneinander für
a) verzweigtes oder unverzweigtes C₁-C₅ Alkyl,
b) substituiertes oder unsubstituiertes Aryl,
c) substituiertes oder unsubstituiertes Alkylaryl oder
d) substituiertes oder unsubstituiertes Heteroaryl
steht,
Z'' für ein Metallkationenequivalent steht, und
wobei n = 0, 1, 2 oder 3 ist.

Bevorzugte Verbindungen der Erfindung gemäß der allgemeinen Formel (I) zeichnen sich dadurch aus, dass
A für
a) verzweigtes oder unverzweigtes C₁-C₅ Alkoxy, oder
b) NH₂,
steht.

Weitere bevorzugte Verbindungen der Erfindung gemäß der allgemeinen Formel (I) zeichnen sich dadurch aus, dass
A für
a) Methoxy,
b) Ethoxy, oder
c) NH₂,
steht.

Bevorzugte Verbindungen der Erfindung gemäß der allgemeinen Formel (I) zeichnen sich dadurch aus, dass
A'' für
Methoxy
steht.

Bevorzugte Verbindungen der Erfindung gemäß der allgemeinen Formel (I) zeichnen sich dadurch aus, dass
A'' für
NH₂
steht.

Bevorzugte Verbindungen der Erfindung gemäß der allgemeinen Formel (I) zeichnen sich dadurch aus, dass
A'' für
Ethoxy
steht.

Bevorzugte Verbindungen der Erfindung gemäß der allgemeinen Formel (III) zeichnen sich dadurch aus, dass
G" für
a) verzweigtes oder unverzweigtes O-C₁-C₅ Alkyl,
b) verzweigtes oder unverzweigtes O-C₂-C₅ Alkenyl,
c) verzweigtes oder unverzweigtes O-C₁-C₅ Alkyl-(O-C₁-C₄ alkyl)ₙ-O-C₁-C₄ alkyl, oder
e) Triphenylmethoxy
steht.

Weiter bevorzugte Verbindungen der Erfindung gemäß der allgemeinen Formel (III) zeichnen sich dadurch aus, dass
G" für
a) Methoxy,
b) Ethoxy,
c) tert-Butoxy,a
steht.

Besonders bevorzugte Verbindungen der Erfindung gemäß der allgemeinen Formel (III) zeichnen sich dadurch aus, dass
G" für
a) Methoxy
steht.

Bevorzugte Verbindungen der Erfindung gemäß der allgemeinen Formel (III) zeichnen sich dadurch aus, dass
R³ und R⁴ für
a) Wasserstoff,
b) verzweigtes oder unverzweigtes E-C₂-C₄ Alkoxy,
c) verzweigtes oder unverzweigtes E-C₃-C₅ Alkyl,
d) verzweigtes oder unverzweigtes E-C₃-C₅ Alkenyl,
e) verzweigtes oder unverzweigtes E-C₃-C₅ Alkinyl, oder
f) verzweigtes oder unverzweigtes C₁-C₅ Alkyl
steht, mit der Maßgabe, dass genau einer der Substituenten R³ oder R⁴ ein E beinhaltet und der jeweils andere Substituent Wasserstoff ist, mit der Maßgabe dass R³ nicht Wasserstoff ist.

Bevorzugte Verbindungen der Erfindung gemäß der allgemeinen Formel (III) zeichnen sich dadurch aus, dass
R³ und R⁴ für
a) Wasserstoff,
b) verzweigtes oder unverzweigtes E-C₂ Alkoxy,
c) verzweigtes oder unverzweigtes E-C₃ Alkyl,
d) verzweigtes oder unverzweigtes E-C₃ Alkenyl, oder
e) verzweigtes oder unverzweigtes E-C₃ Alkinyl,
f) Hydroxyl,
g) verzweigtes oder unverzweigtes C₁-C₅ Alkyl oder
h) verzweigtes oder unverzweigtes C₁-C₅ Alkoxy
steht,
steht, mit der Maßgabe, dass genau einer der Substituenten R³ oder R⁴ ein E beinhaltet und der jeweils andere Substituent kein E beinhaltet,

Ein weiterer besonderer Gegenstand der Erfindung sind Verbindungen der allgemeinen Formel III worin
R³ für
a) verzweigtes oder unverzweigtes E-C₂ Alkoxy,
b) verzweigtes oder unverzweigtes E-C₃ Alkyl,
c) verzweigtes oder unverzweigtes E-C₃ Alkenyl, oder
d) verzweigtes oder unverzweigtes E-C₃ Alkinyl
steht.

Unverzweigtes E-C₂ Alkoxy ist E-Ethoxy.
Unverzweigtes E-C₃ Alkyl ist E-Propyl.
Unverzweigtes E-C₃ Alkenyl ist E-Propenyl.
Unverzweigtes E-C₃ Alkinyl ist E-Propinyl.

Ein weiterer besonderer Gegenstand der Erfindung sind Verbindungen der allgemeinen Formel III worin
R³ für E-Ethoxy oder E-Propyl und R⁴ für Wasserstoff steht.

Bevorzugte Verbindungen der Erfindung gemäß der allgemeinen Formel (III) zeichnen sich dadurch aus, dass
E für
a) Chloro,
b) Bromo,
c) Methansulfonyloxy,
d) Trifluormethansulfonyloxy,
e) Tosyloxy oder
f) Iodo
steht.

Weitere bevorzugte Verbindungen der Erfindung gemäß der allgemeinen Formel (III) zeichnen sich dadurch aus, dass
E für
a) Bromo,
b) Methansulfonyloxy,
d) Trifluormethansulfonyloxy,
steht.

Besonders bevorzugte Verbindungen der Erfindung gemäß der allgemeinen Formel (III) zeichnen sich dadurch aus, dass
E für
a) Bromo, oder
b) Methansulfonyloxy,
   steht.

Bevorzugte Verbindungen der Erfindung gemäß der allgemeinen Formel (III) zeichnen sich dadurch aus, dass
Q' für
a) N(H)-tert-Butoxycarbonyl,
b) N(H)-Benzyloxycarbonyl,
c) N(H)-Trityl, oder
d) 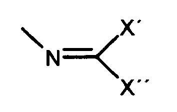
steht.

Weiter bevorzugte Verbindungen der Erfindung gemäß der allgemeinen Formel (III) zeichnen sich dadurch aus, dass
Q' für
a) N(H)-tert-Butoxycarbonyl, oder
b) 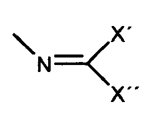
steht.

Bevorzugte Verbindungen der Erfindung gemäß der Formel (III) zeichnen sich dadurch aus, dass
L" für
a) verzweigtes oder unverzweigtes C₁-C₅ Alkyl,
b) verzweigtes oder unverzweigtes C₂-C₅ Alkenyl, oder
c) verzweigtes oder unverzweigtes C₂-C₅ Alkinyl

Bevorzugte Verbindungen der Erfindung gemäß der Formel (III) zeichnen sich dadurch aus, dass
L" für
a) Methyl,
b) Ethyl,
c) Propyl,
d) iso-Propyl,
e) -C₂H₄-OMe, oder
f) -C₂H₄-O-C₂H₄-OMe
steht.

Weiter bevorzugte Verbindungen der Erfindung gemäß der Formel (III) zeichnen sich dadurch aus, dass
L" für
a) Methyl, oder
b) Ethyl
steht.

Bevorzugte Verbindungen der Erfindung gemäß der Formel (III) zeichnen sich dadurch aus, dass
X' and X'' unabhängig voneinander für
a) verzweigtes oder unverzweigtes C₁-C₅ Alkyl,
b) substituiertes oder unsubstituiertes Aryl, oder
c) Aralkyl
steht.

Weiter bevorzugte Verbindungen der Erfindung gemäß der Formel (III) zeichnen sich dadurch aus, dass
X' und X'' unabhängig voneinander für
a) verzweigtes oder unverzweigtes C₁-C₅ Alkyl, und
b) substituiertes oder unsubstituiertes Aryl
steht.

Besonders bevorzugte Verbindungen der Erfindung gemäß der Formel (III) zeichnen sich dadurch aus, dass
X' und X'' für Phenyl bzw. für in 2-Position substituiertes Phenyl steht.

Ebenfalls bevorzugte Verbindungen der Erfindung gemäß der Formel (III) zeichnen sich dadurch aus, dass Z' ist ausgewählt aus der Gruppe Na⁺, K⁺, Ca²⁺ und Mg²⁺.
Bevorzugtes Z' ist Na⁺.

In einem weiteren Aspekt bezieht sich somit die vorliegende Erfindung auf Verwendung von Verbindungen der Formel (IV) zur Herstellung von Verbindungen der Formel (I) oder (II): 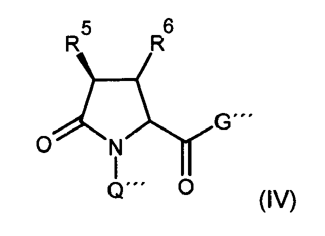 G''' für
a) verzweigtes oder unverzweigtes O-C₁-C₅ Alkyl,
b) verzweigtes oder unverzweigtes O-C₂-C₅ Alkenyl,
c) verzweigtes oder unverzweigtes O-C₁-C₅ Alkyl-(O-C₁-C₄ alkyl)ₙ-O-C₁-C₄ alkyl,
d) verzweigtes oder unverzweigtes O-C₂-C₅ Alkinyl, oder
e) Triphenylmethoxy
steht,
R⁵ und R⁶ für
a) Wasserstoff
b) Hydroxyl,
c) verzweigtes oder unverzweigtes C₁-C₅ Alkyl,
d) verzweigtes oder unverzweigtes C₁-C₅ Alkoxy oder
e) R⁷-E'
   steht, mit der Maßgabe, dass genau einer der Substituenten R⁵ oder R⁶ ein E' beinhaltet und der jeweils andere Substituent kein E' beinhaltet, mit der Maßgabe
   dass R⁵ nicht Wasserstoff ist,
E'für
a) Chloro,
b) Bromo,
c) Methansulfonyloxy,
d) Trifluormethansulfonyloxy,
e) Nonafluorbutyloxy,
f) Tosyloxy oder
g) Iodo
steht,
R⁷ für
a) verzweigtes oder unverzweigtes C₁-C₅ Alkoxy,
b) verzweigtes oder unverzweigtes C₁-C₅ Alkyl,
c) verzweigtes oder unverzweigtes C₂-C₅ Alkenyl, oder
d) verzweigtes oder unverzweigtes C₂-C₅ Alkinyl
steht,
Q''' für
a) N-tert-Butoxycarbonyl,
b) N-Allyloxycarbonyl,
c) N-Benzyloxycarbonyl,
d) N-Ethoxycarbonyl,
e) N-Methoxycarbonyl,
f) N-Propoxycarbonyl,
g) N-2,2,2-Trichlorethoxycarbonyl,
h) Wasserstoff,
i) N-1-Methyl-1-phenyl-ethoxycarbonyl,
j) N-1-Methyl-1-(4-biphenylyl)-ethoxycarbonyl,
k) N-cyclobutylcarbonyl,
l) N-1-Methylcyclobutylcarbonyl,
m) N-Vinylcarbonyl,
n) N-Allylcarbonyl,
o) N-Adamantylcarbonyl,
p) N-Diphenylmethylcarbonyl,
q) N-Cinnamylcarbonyl,
r) N-Formyl, oder
s) N-Benzoyl,
t) N(H)-Trityl,
u) N(H)-p-Methoxyphenyl-diphenylmethyl,
v) N(H)-di-(p-Methoxyphenyl)-phenylmethyl,
w) 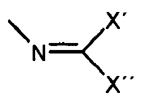 oder
x) N-(tert-Butoxycarbonyl)₂,
   steht,
   X' and X'' unabhängig voneinander für
   a) verzweigtes oder unverzweigtes C₁-C₅ Alkyl,
   b) substituiertes oder unsubstituiertes Aryl,
   c) substituiertes oder unsubstituiertes Alkylaryl oder
   d) substituiertes oder unsubstituiertes Heteroaryl steht, und
wobei n = 0, 1, 2 oder 3 ist.

Besonders bevorzugte Verbindungen der Erfindung gemäß der Formel (IV) zeichnen sich dadurch aus, dass
G''' für
a) verzweigtes oder unverzweigtes O-C₁-C₅ Alkyl,
b) verzweigtes oder unverzweigtes O-C₂-C₅ Alkenyl,
c) verzweigtes oder unverzweigtes O-C₁-C₅ Alkyl-(O-C₁-C₄ alkyl)ₙ-O-C₁-C₄ alkyl, oder
e) Triphenylmethoxy
steht.

Weiter bevorzugte Verbindungen der Erfindung gemäß der Formel (IV) zeichnen sich dadurch aus, dass
G''' für
a) Methoxy,
b) Ethoxy,
c) tert-Butoxy,a
steht.

Besonders bevorzugte Verbindungen der Erfindung gemäß der Formel (IV) zeichnen sich dadurch aus, dass
G''' für
a) Methoxy
steht.

Bevorzugte Verbindungen der Erfindung gemäß der Formel (IV) zeichnen sich dadurch aus, dass
E' für
a) Bromo,
b) Methansulfonyloxy,
d) Trifluormethansulfonyloxy, oder
c) Trifluormesyloxy
   steht.

Weiter bevorzugte Verbindungen der Erfindung gemäß der Formel (IV) zeichnen sich dadurch aus, dass
E'für
a) Chloro,
b) Bromo,
c) Methansulfonyloxy,
d) Trifluormethansulfonyloxy,
e) Tosyloxy oder
f) Iodo
steht.

Besonders bevorzugte Verbindungen der Erfindung gemäß der Formel (IV) zeichnen sich dadurch aus, dass
E' für
a) Bromo, oder
b) Methansulfonyloxy
steht.

Bevorzugte Verbindungen der Erfindung gemäß der Formel (IV) zeichnen sich dadurch aus, dass
Q' für
a) N(H)-tert-Butoxycarbonyl,
b) N(H)-Benzyloxycarbonyl,
c) N(H)-Trityl, oder
d) 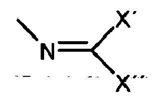
steht.

Weiter bevorzugte Verbindungen der Erfindung gemäß der Formel (IV) zeichnen sich dadurch aus, dass
Q' für
a) N(H)-tert-Butoxycarbonyl, oder
b) 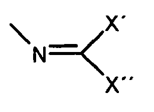 steht.

Bevorzugte Verbindungen der Erfindung gemäß der Formel (III) zeichnen sich dadurch aus, dass
X' and X'' unabhängig voneinander für
a) verzweigtes oder unverzweigtes C₁-C₅ Alkyl,
b) substituiertes oder unsubstituiertes Aryl, oder
c) Aralkyl
steht.

Weiter bevorzugte Verbindungen der Erfindung gemäß der Formel (III) zeichnen sich dadurch aus, dass
X' und X'' unabhängig voneinander für
a) verzweigtes oder unverzweigtes C₁-C₅ Alkyl, und
b) substituiertes oder unsubstituiertes Aryl
steht.

Besonders bevorzugte Verbindungen der Erfindung gemäß der Formel (III) zeichnen sich dadurch aus, dass
X' und X'' für Phenyl bzw. für in 2-Position substituiertes Phenyl steht.

R⁵ und R⁶ für
a) Wasserstoff,
b) Hydroxyl,
c) verzweigtes oder unverzweigtes C₃-C₅ Alkyl,
d) verzweigtes oder unverzweigtes C₃-C₅ Alkoxy
e) R⁷-E'
steht, mit der Maßgabe, dass genau einer der Substituenten R⁵ oder R⁶ ein E' beinhaltet und der jeweils andere Substituent kein E' beinhaltet,
E' für
a) Chloro,
b) Bromo,
c) Mesyloxy,
d) Trifluoromethylsulfonyloxy,
e) Nonafluorbutyloxy,
f) Tosyloxy oder
g) Iodo
steht,
R⁷ für
a) verzweigtes oder unverzweigtes C₁-C₅ Alkoxy,
b) verzweigtes oder unverzweigtes C₁-C₅ Alkyl,
c) verzweigtes oder unverzweigtes C₂-C₅ Alkenyl, oder
d) verzweigtes oder unverzweigtes C₂-C₅ Alkinyl
steht,

In einem weiteren Aspekt bezieht sich die vorliegende Erfindung auf einen Bildgebungs-Kit enthaltend Verbindungen der allgemeinen Formel III oder IV.

In einem weiteren Aspekt bezieht sich die vorliegende Erfindung auf Pharmazeutische Zusammensetzungen enthaltend Verbindungen der allgemeinen Formel I, II, III oder IV und geeignete pharmazeutische Träger Substanzen.

Verbindungen nach Formel I oder II **dadurch gekennzeichnet dass** die Verbindungen für Bildgebung in einem Dosisbereich von 37 - 600 MBq geeignet sind.

Bevorzugte Verbindungen nach Formel I oder II **dadurch gekennzeichnet dass** die Verbindungen in einem Dosisbereich von 150 MBq - 370 MBq besonders geeignet sind.

Das Verfahren zur Herstellung der erfindungsgemäßen Verbindungen der allgemeinen Formel (I) oder (II) zeichnet sich dadurch aus, dass die Mehrzahl der Verbindungen nach Formel (I) oder (II), aus einer Verbindung der Verbindungen der allgemeinen Formel (IV) nach Einführung des ¹⁸F Isotops entstehen kann.

Die vorliegende Erfindung betrifft Verbindungen der allgemeinen Formel (IV).

Bevorzugt für die Einführung des ¹⁸F Isotops sind
4,7,13,16,21,24-Hexaoxa-1,10-diazabicyclo[8.8.8]-hexacosane K18F (Kronenethersalz Kryptofix K18F),
K¹⁸F,
H¹⁸F,
KH¹⁸F₂,
Cs¹⁸F,
Na¹⁸F oder
¹⁸F tetraalkylammonium salz (z.b [F-18] tetrabutylammonium fluoride).

Die vorliegende Erfindung betrifft Verbindungen der allgemeinen Formel (I) oder (II) und Verfahren wobei das Fluorisotop ¹⁸F verwendet wird.

Wenn ein oder mehrere chirale Zentren in einer Verbindung des vorliegenden Erfindungsgegenstandes der Formel (I), Formel (II), Formel (III), oder Formel (IV) vorhanden ist bzw. sind, so sollen alle Formen dieses Isomers, einschließlich beider Enantiomere und aller möglichen Diastereomere, hierin beinhaltet sein, mit der Maßgabe, dass der Substituent R¹ - R⁶ in S-Konfiguration vorliegt. In solchen Fällen, in denen eine Kohlenstoff-Kohlenstoff-Doppelbindung vorliegt, so sind beide Isomere "cis" und "trans" Teil der vorliegenden Erfindung. In solchen Fällen, in denen tautomere Formen vorliegen können, wie z. Bsp. Keto-enol Tautomerie, sind alle tautomeren Formen in der vorliegenden Erfindung beinhaltet, wobei diese Formen im Gleichgewicht oder bevorzugt in einer Form vorliegen können.

Die Verbindungen der allgemeinen Formel I oder II und ihre bevorzugten Ausführungsformen werden verwendet als Arzneimittel.

Die erfindungsgemäßen Verbindungen der allgemeinen Formel I oder II und ihre bevorzugten Ausführungsformen werden verwendet in der Diagnose/ Bildgebung von physiologischen oder pathologischen Zuständen.
Bevorzugt finden diese Verbindungen Anwendung in der nicht-invasiven PET-basierten Diagnose am menschlichen oder tierischen Körper.
Besonders bevorzugt finden die erfindungsgemäßen Verbindungen der allgemeinen Formel I oder II und ihre bevorzugten Ausführungsformen Anwendung in der Diagnose von Tumorerkrankungen. Beispiele für solche Tumorerkrankungen sind Malignome des Gastrointestinal- oder Kolorektaltraktes, Leber-, Pankreas-, Nieren-, Blasen-, Schilddrüsen-, Prostata-, Endometrium-, Ovar-, Testes-, Melanom-, kleinzelliges und nicht-kleinzelliges Bronchialkarzinom, dysplastisches orales Mucosa-Karzinom, invasiver Oral-Krebs; Brustkrebs, einschließlich hormonabhängigem und hormonunabhängigem Brustkrebs, Plattenepithelkarzinom, neurologische Krebserkrankungen einschließlich Neuroblastom, Gliom, Astrocytom, Osteosarcom, Meningiom; Weichteilsarkom; Hämangioam und endokrine Tumore, einschließlich Hypophysenadenome, Chromocytome, Paragangliome, haematologische Tumorerkrankungen einschließlich Lymphoma and Leukämien; oder Metastasen einer der oben genannten Tumoren.
Die erfindungsgemäßen Verbindungen der allgemeinen Formel I oder II und ihre bevorzugten Ausführungsformen werden verwendet zur Herstellung eines Arzneimittels für die Diagnose von Tumorerkrankungen. Beispiele für solche Tumorerkrankungen sind Malignome des Gastrointestinal- oder Kolorektaltraktes, Leber-, Pankreas-, Nieren-, Blasen-, Schilddrüsen-, Prostata-, Endometrium-, Ovar-, Testes-, Melanom-, kleinzelliges und nicht-kleinzelliges Bronchialkarzinom, dysplastisches orales Mucosa-Karzinom, invasiver Oral-Krebs; Brustkrebs, einschließlich Hormon-abhängigem und Hormon-unabhängigem Brustkrebs, Plattenepithelkarzinom, neurologische Krebserkrankungen einschließlich Neuroblastom, Gliom, Astrocytom, Osteosarcom, Meningiom, Weichteilsarkom; Hämangioam und endokrine Tumore, einschließlich Hypophysenadenome, Chromocytome, Paragangliome, haematologische Tumorerkrankungen einschließlich Lymphoma and Leukämien; oder Metastasen einer der oben genannten Tumoren.

Die Erfindung betrifft pharmazeutische Präparate, welche mindestens eine Verbindung der Formel I, II, III oder IV sowie einen pharmazeutisch verträglichen Träger enthalten.

Zur Verwendung der Verbindungen der Formel I, II, III oder IV als Arzneimittel werden diese in die Form eines pharmazeutischen Präparats gebracht, welches neben dem Wirkstoff für die enterale oder parenterale Applikation geeignete pharmazeutische, organische oder anorganische inerte Trägermaterialien, wie zum Beispiel, Wasser, Gelatine, Gummi arabicum, Milchzucker, Stärke, Magnesiumstearat, Talk, pflanzliche Öle, Polyalkylenglykole usw. enthält.

Die Erfindung betrifft eine Einrichtung (Kit) enthaltend mindestens eine Verbindung der Formel I, II, III, oder IV.

Erfindungsgemäße Verbindungen, bei denen das [F-18]-Isotop über eine Methylengruppe in 4-Position des Glutaminsäuregerüstes positioniert ist, wie z. B. bei 4S-[F-18]Fluoromethyl-glutaminsäure **1**, lassen sich wie in Schema 7 dargestellt, herstellen. So gelingt beispielshalber die saure Abspaltung der Schutzgruppen der Verbindung **2**, um die erfindungsgemäße Verbindung 4S-[F-18]Fluoromethyl-glutaminsäure **1** zu erhalten.

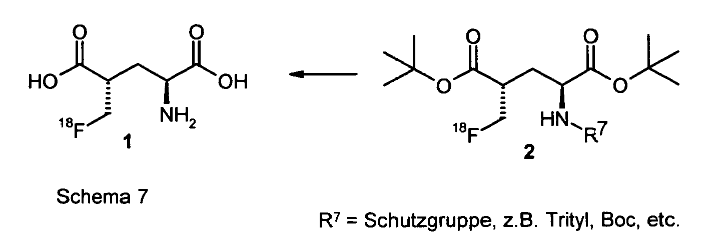

Dabei können verschiedene organische (z. B. Trifluoressigsäure), vor allem aber anorganische Säuren wie z. B. Bromwasserstoffsäure, Salzsäure, Schwefelsäure, Perchlorsäure oder Phosphorsäure zum Einsatz kommen. Die Reinigung der erfindungsgemäßen Verbindung **2** nach Formel (I) ist per HPLC möglich, wobei verschiedene Reinigungsschritte prinzipiell vorgeschaltet und nachgeschaltet sein können, wie z. B. die Reinigung über eine RP-C18 Kartusche oder andere Trennmaterialien.

Die radiochemische Fluorierung von Tosylat **3**, dessen Synthese analog der in der Literatur beschriebenen Methode (Chem. Pharm. Bull., 17, 5, (1969), 879-885) aus **4** (Tetrahedron, 45, 5, (1989) 1453-1464) erfolgte, zum [F-18]-markierten Glutaminsäurederivat **2** ist nach den dem Fachmann bekannten Methoden durchführbar (s. Schema 8).

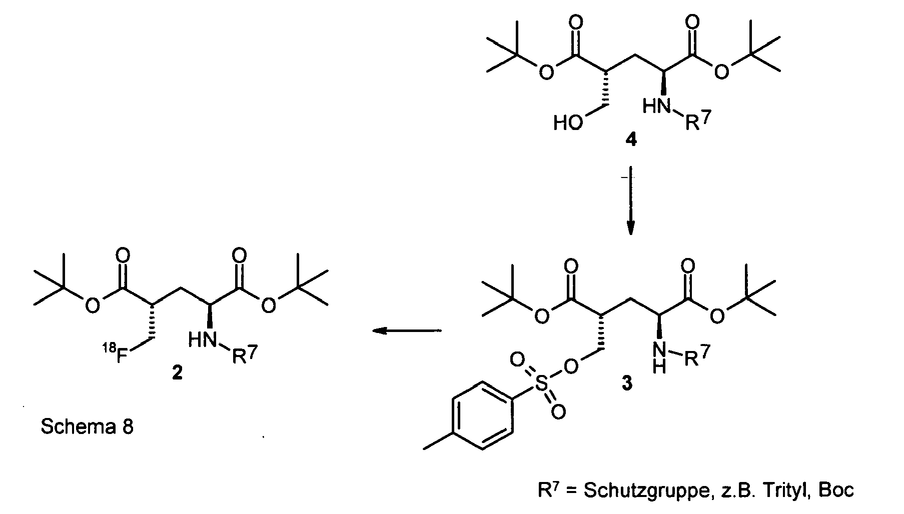

Dabei kann Verbindung **3** in Gegenwart einer Base wie zum Beispiel Tetra-alkyl ammonium und Tetra-alkyl-phosphonium-carbonat und Kaliumcarbonat etc. mit der entsprechenden [F-181-Fluoridlösung umgesetzt werden. Die Reaktion läuft bevorzugt bei erhöhten Temperaturen ab. Der Zusatz von Kronenethern, wie z. B. Kryptofix (K2.2.2) kann die Reaktion positiv beeinflussen, besonders in Kombination mit K₂CO₃ als katalysierende Base. Mögliche Lösungsmittel sind bevorzugt aprotisch, aber auch protische Lösungsmittel oder aber aprotische Lösungsmittelzusätze, wie z. B. Wasser, können zur Anwendung kommen. Üblicherweise werden für die radiochemische Fluorierung mit [F-18]-Fluoridanionen Acetonitril, Dimethylsulfoxid oder Dimethylformamid als optimale Lösungsmittel angewandt. Verbindung **2** muss üblicherweise keiner Reinigung unterzogen werden, sondern kann umgehend mit den für die Umsetzung von **2** nach **1** beschriebenen Methoden behandelt werden. Eine Reinigung der Verbindung **2** ist aber prinzipiell möglich, bevorzugt mittels einer präparativen HPLC mit einer unpolaren Phase, wie z. B. RP C-18.

Erfindungsgemäße Verbindungen, bei denen das [F-18]-Isotop über eine Alkoxygruppe in 4S-Konfiguration in 4-Position des Glutaminsäuregerüstes positioniert ist, wie z. B. bei 4-S-(2-[F-18]Fluoroethoxy)-glutaminsäure **5,** lassen sich wie in Schema 9 dargestellt, herstellen. So gelingt beispielshalber die saure Abspaltung der Schutzgruppen der Verbindung **6** oder **7** um die erfindungsgemäße Verbindung 4-S-[F-18]Fluoroethoxy-glutaminsäure **5** zu erhalten.

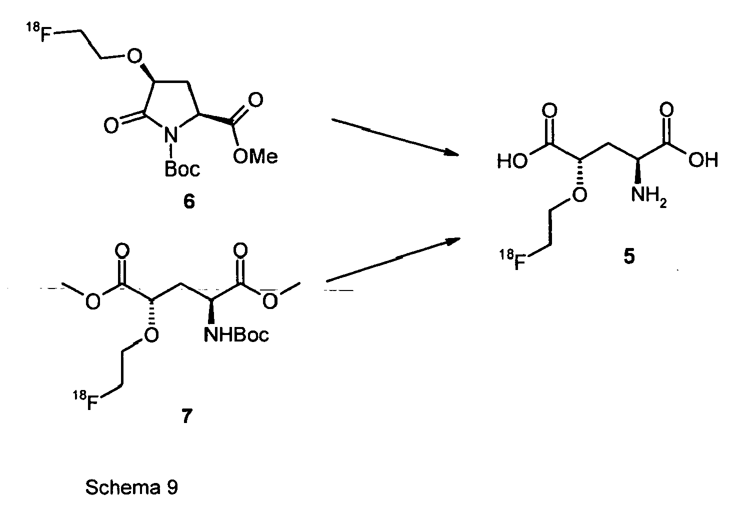

Dabei können verschiedene organische (z. B. Trifluoressigsäure), vor allem aber anorganische Säuren wie z. B. Bromwasserstoffsäure, Salzsäure, Schwefelsäure, Perchlorsäure oder Phosphorsäure zum Einsatz kommen. Weiterhin ist auch eine basische Ringöffnung von **6** möglich mit Lithiumhydroxid, Natriumhydroxid, Kaliumhydroxid etc. (S. Baker et al. Tetrahedron Lett. 1998, 39, 2815-2818.).
Die Reinigung der erfindungsgemäßen Verbindung **5** nach Formel (I) ist per HPLC möglich, wobei verschiedene Reinigungsschritte prinzipiell vorgeschaltet und nachgeschaltet sein können, wie z. B. die Reinigung über eine RP-C18 Kartusche oder andere Trennmaterialien.

Die radiochemische Fluorierung von Tosylat **8** dessen Synthese analog der in der Literatur beschriebenen Methode (N. Sharma et al. Tetrahedron Lett. 2004, 45, 1403-1406.) aus **9** erfolgte, zum [F-18]-markierten S-Glutaminsäurederivat **6** ist nach den dem Fachmann bekannten Methoden durchführbar (s. Schema 10). 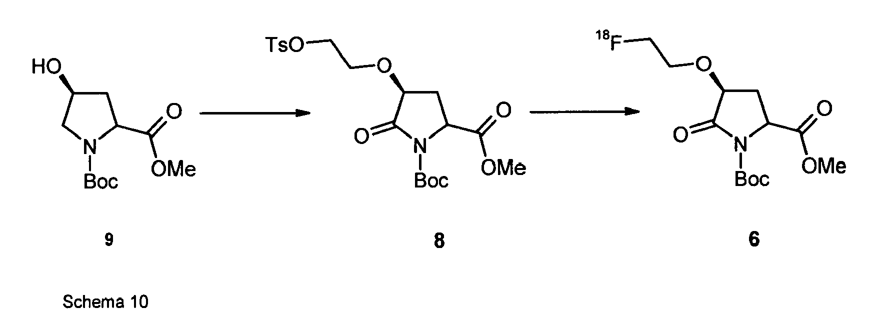 Dabei kann Verbindung **6** in Gegenwart einer Base wie zum Beispiel Tetra-alkyl ammonium und Tetra-alkyl-phosphonium-carbonat und Kaliumcarbonat etc. mit der entsprechenden [F-18]-Fluoridlösung umgesetzt werden. Die Reaktion läuft bevorzugt bei erhöhten Temperaturen ab. Der Zusatz von Kronenethern, wie z. B. Kryptofix (K2.2.2) kann die Reaktion positiv beeinflussen, besonders in Kombination mit K₂CO₃ als katalysierende Base. Mögliche Lösungsmittel sind bevorzugt aprotisch, aber auch protische Lösungsmittel oder aber aprotische Lösungsmittelzusätze, wie z. B. Wasser, können zur Anwendung kommen. Üblicherweise werden für die radiochemische Fluorierung mit [F-18]-Fluoridanionen Acetonitril, Dimethylsulfoxid oder Dimethylformamid als optimale Lösungsmittel angewandt. Verbindung **6** muss üblicherweise keiner Reinigung unterzogen werden, sondern kann umgehend mit den für die Umsetzung von **6** nach **6** beschriebenen Methoden behandelt werden. Eine Reinigung der Verbindung **6** ist aber prinzipiell möglich, bevorzugt mittels einer präparativen HPLC mit einer unpolaren Phase, wie z. B. RP C-18. Außerdem ist eine Reinigung mittels Kartuschen möglich.

Die radiochemische Fluorierung von Tosylat **10** dessen Synthese analog der in der Literatur beschriebenen Methode (X. Zhang Tetrahedron Lett. 2001, 42, 5335-5338.) aus **8** erfolgte, zum [F-18]-markierten Glutaminsäurederivat **7** ist nach den dem Fachmann bekannten Methoden durchführbar (s. Schema 11).

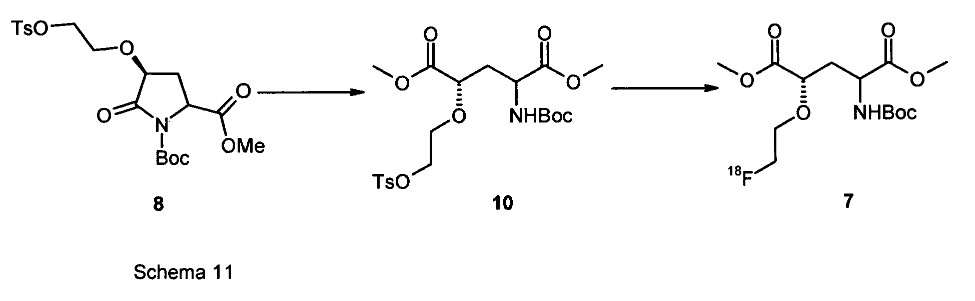

Dabei kann Verbindung **10** in Gegenwart einer Base wie zum Beispiel Tetra-alkyl ammonium und Tetra-alkyl-phosphonium-carbonat und Kaliumcarbonat etc. mit der entsprechenden [F-18]-Fluoridlösung umgesetzt werden. Die Reaktion läuft bevorzugt bei erhöhten Temperaturen ab. Der Zusatz von Kronenethern, wie z. B. Kryptofix (K2.2.2) kann die Reaktion positiv beeinflussen, besonders in Kombination mit K₂CO₃ als katalysierende Base. Mögliche Lösungsmittel sind bevorzugt aprotisch, aber auch protische Lösungsmittel oder aber aprotische Lösungsmittelzusätze, wie z. B. Wasser, können zur Anwendung kommen. Üblicherweise werden für die radiochemische Fluorierung mit [F-18]-Fluoridanionen Acetonitril, Dimethylsulfoxid oder Dimethylformamid als optimale Lösungsmittel angewandt. Verbindung **7** muss üblicherweise keiner Reinigung unterzogen werden, sondern kann umgehend mit den für die Umsetzung von **7** nach **5** beschriebenen Methoden behandelt werden. Eine Reinigung der Verbindung **7** ist aber prinzipiell möglich, bevorzugt mittels einer präparativen HPLC mit einer unpolaren Phase, wie z. B. RP C-18. Außerdem ist eine Reinigung mittels Kartuschen möglich.

Als Ausgangsverbindung für die radiochemische Fluorierung zu **6** eignet sich auch das Bromid **8a,** das aus **9** in zwei Stufen erhalten werden kann: durch Alkylierung von **9** mittels 1,2-Dibromethan zum Bromethoxy-pyrrolidin-derivat **8b** und anschließender Oxidation, z.B. mittels Ruthenium(III)-verbindungen, wie in den Beispielen **3a** und **3b** beschrieben.

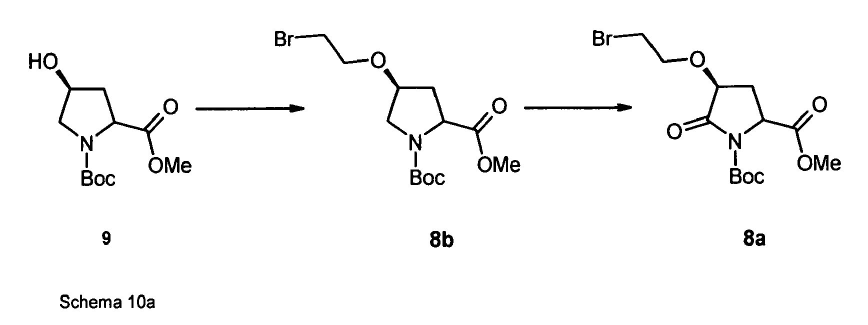

Die Synthese von F-19 Referenzverbindungen **11, 12** und **13** kann wie in Schema 12 dargestellt erfolgen. 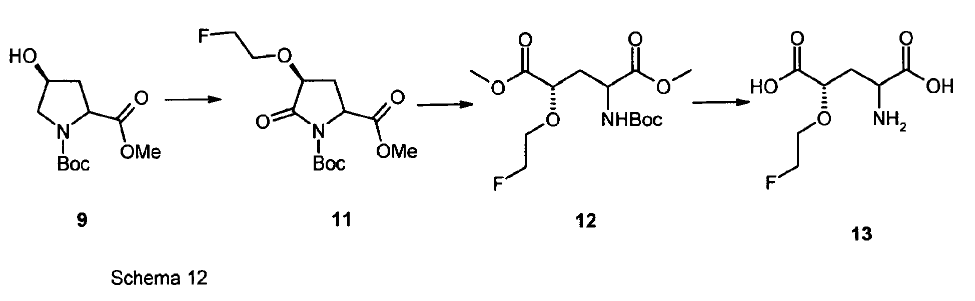 **11** kann durch Alkylierung und Oxidation des Hydroxyprolinderivats **9** erhalten werden. Vorteilhaft erweist sich bei der Darstellung von F-19 Referenzverbindungen auch die Darstellung der Fluoride aus den analogen Hydroxyverbindungen mittels DAST (Diethylaminoschwefeltrifluorid) nach den dem Fachmann bekannten Verfahren wie z.B. in Beispiel **1d** beschrieben.
Durch Ringöffnung des Pyroglutaminderivats **26** wird die offenkettige Referenzverbindung **12** erhalten. Die saure Abspaltung der Schutzgruppen führt zum Glutaminsäurederivat **13.**

Erfindungsgemäße Verbindungen, bei denen das [F-18]-Isotop über eine Alkylgruppe in 4-Position des Glutaminsäuregerüstes positioniert ist, wie z. B. bei 4-[F-18]Fluoropropylglutaminsäure **14** oder 4-[F-18]Fluorobutyl-glutaminsäure **15** lassen sich wie in Schema 13 dargestellt, herstellen. So gelingt beispielshalber die saure Abspaltung der Schutzgruppen der Verbindungen **16** und **17,** um die erfindungsgemäßen Verbindung 4-[F-18]Fluoropropylglutaminsäure **14** oder 4-[F-18]Fluorobutyl-glutaminsäure **15** zu erhalten.

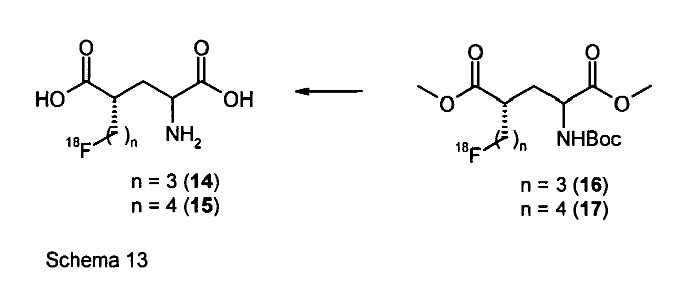

Dabei können verschiedene organische (z. B. Trifluoressigsäure), vor allem aber anorganische Säuren wie z. B. Bromwasserstoffsäure, Salzsäure, Schwefelsäure, Perchlorsäure oder Phosphorsäure zum Einsatz kommen. Die Reinigung der erfindungsgemäßen Verbindung **14** und **15** nach Formel (I) ist per HPLC möglich, wobei verschiedene Reinigungsschritte prinzipiell vorgeschaltet und nachgeschaltet sein können, wie z. B. die Reinigung über eine RP-C18 Kartusche oder andere Trennmaterialien.

Die radiochemische Fluorierung von Bromid **18** oder Tosylat 19, deren Synthese analog der in der Literatur beschriebenen Methode (S. Hanessian, et al. J. Org. Chem. 2005, 70, 5070-5085) aus **20** erfolgte, zu den [F-18]-markierten Glutaminsäurederivaten **16** und **17** ist nach den dem Fachmann bekannten Methoden durchführbar (s. Schema 14).

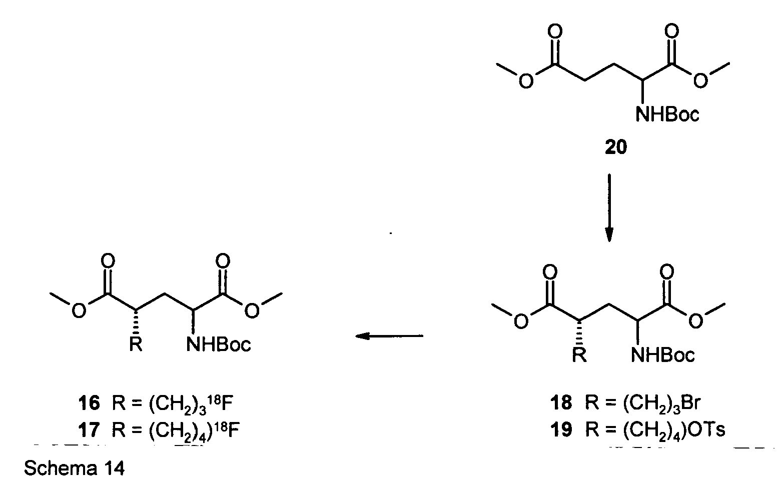

Dabei können die Verbindungen **18** und **19** in Gegenwart einer Base wie zum Beispiel Tetra-alkyl ammonium und Tetra-alkyl-phosphonium-carbonat und Kaliumcarbonat etc. mit der entsprechenden [F-18]-Fluoridlösung umgesetzt werden. Die Reaktion läuft bevorzugt bei erhöhten Temperaturen ab. Der Zusatz von Kronenethern, wie z. B. Kryptofix (K2.2.2) kann die Reaktion positiv beeinflussen, besonders in Kombination mit K₂CO₃ als katalysierende Base. Mögliche Lösungsmittel sind bevorzugt aprotisch, aber auch protische Lösungsmittel oder aber aprotische Lösungsmittelzusätze, wie z. B. Wasser, können zur Anwendung kommen. Üblicherweise werden für die radiochemische Fluorierung mit [F-18]-Fluoridanionen Acetonitril, Dimethylsulfoxid oder Dimethylformamid als optimale Lösungsmittel angewandt. Die Verbindungen **16** und **17** müssen üblicherweise keiner Reinigung unterzogen werden, sondern können umgehend mit den für die Umsetzung von **16** nach **14** bzw. **17** nach **15** beschriebenen Methoden behandelt werden. Eine Reinigung der Verbindungen **16** bzw. **17** ist aber prinzipiell möglich, bevorzugt mittels einer präparativen HPLC mit einer unpolaren Phase, wie z. B. RP C-18.

Die Synthese der F-19 Referenzverbindungen **21** und **22** kann durch Alkylierung des Glutaminsäurederivats **20** erfolgen (Schema 15). Die Verbindung **20** kann auch mit Iodiden, vorzugsweise mit Diiodiden analog Beispiel **2a** alkyliert werden. Man erhält dann in einem Schritt aus kommerziell verfügbarem Glutaminsäurederivat **20** einen zur radiochemischen Fluorierung geeigneten Präkursor.

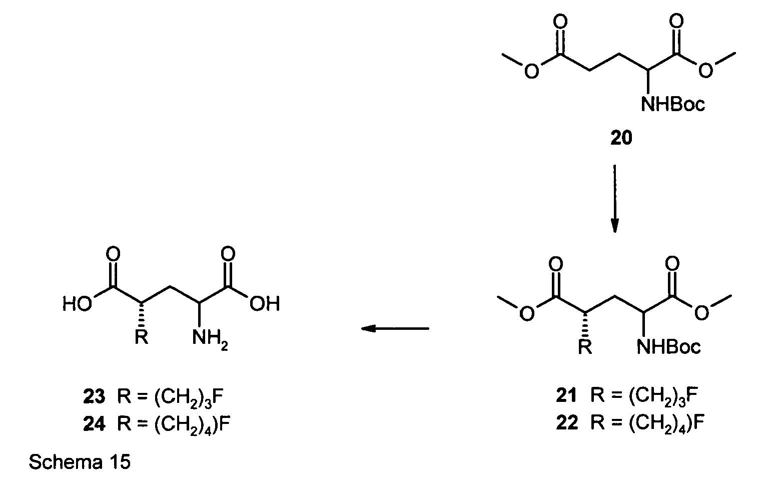

Die Abspaltung der Schutzgruppen führt zu den Fluoralkylierten Glutaminsäurederivaten **23** und **24.**

Es sollen jeweils die S-Formen der chirale Zentren in C-3 oder/ und C-4-Position einer Verbindung des vorliegenden Erfindungsgegenstandes der Formel (I), Formel (II), Formel (III), Formel (IV) oder Formel (V) beinhaltet sein. In solchen Fällen, in denen eine Kohlenstoff-Kohlenstoff-Doppelbindung vorliegt, so sind beide Isomere "cis" und "trans" Teil der vorliegenden Erfindung. In solchen Fällen, in denen tautomere Formen vorliegen können, wie z. B. Keto-enol Tautomerie, sind alle tautomeren Formen in der vorliegenden Erfindung beinhaltet, wobei diese Formen im Gleichgewicht oder bevorzugt in einer Form vorliegen können.

Die Verbindungen der allgemeinen Formel I oder II und ihre bevorzugten Ausführungsformen werden verwendet als Arzneimittel.
Die erfindungsgemäßen Verbindungen der allgemeinen Formel I oder II und ihre bevorzugten Ausführungsformen werden verwendet in der Diagnose von physiologischen oder pathologischen Zuständen.
Bevorzugt finden diese Verbindungen Anwendung in der nicht-invasiven PET-basierten Diagnose am menschlichen oder tierischen Körper.
Besonders bevorzugt finden die erfindungsgemäßen Verbindungen der allgemeinen Formel I oder II und ihre bevorzugten Ausführungsformen Anwendung in der Diagnose von Tumorerkrankungen. Beispiele für solche Tumorerkrankungen sind Malignome des Gastrointestinal- oder Kolorektaltraktes, Leber-, Pankreas-, Nieren-, Blasen-, Schilddrüsen-, Prostata-, Endometrium-, Ovar-, Testes-, Melanom-, kleinzelliges und nicht-kleinzelliges Bronchialkarzinom, dysplastisches orales Mucosa-Karzinom, invasiver Oral-Krebs; Brustkrebs, einschließlich hormonabhängigem und hormonunabhängigem Brustkrebs, Plattenepithelkarzinom, neurologische Krebserkrankungen einschließlich Neuroblastom, Gliom, Astrocytom, Osteosarcom, Meningiom; Weichteilsarkom; Hämangioam und endokrine Tumore, einschließlich Hypophysenadenome, Chromocytome, Paragangliome, haematologische Tumorerkrankungen einschließlich Lymphoma and Leukämien; oder Metastasen einer der oben genannten Tumoren.
Die erfindungsgemäßen Verbindungen der allgemeinen Formel I oder II und ihre bevorzugten Ausführungsformen werden verwendet zur Herstellung eines Arzneimittels für die Diagnose von Tumorerkrankungen. Beispiele für solche Tumorerkrankungen sind Malignome des Gastrointestinal- oder Kolorektaltraktes, Leber-, Pankreas-, Nieren-, Blasen-, Schilddrüsen-, Prostata-, Endometrium-, Ovar-, Testes-, Melanom-, kleinzelliges und nicht-kleinzelliges Bronchialkarzinom, dysplastisches orales Mucosa-Karzinom, invasiver Oral-Krebs; Brustkrebs, einschließlich Hormon-abhängigem und Hormon-unabhängigem Brustkrebs, Plattenepithelkarzinom, neurologische Krebserkrankungen einschließlich Neuroblastom, Gliom, Astrocytom, Osteosarcom, Meningiom, Weichteilsarkom; Hämangioam und endokrine Tumore, einschließlich Hypophysenadenome, Chromocytome, Paragangliome, haematologische Tumorerkrankungen einschließlich Lymphoma and Leukämien; oder Metastasen einer der oben genannten Tumoren.

Die Erfindung betrifft pharmazeutische Präparate, welche mindestens eine Verbindung der Formel I oder II sowie einen pharmazeutisch verträglichen Träger enthalten.

Zur Verwendung der Verbindungen der Formel I oder II als Arzneimittel werden diese in die Form eines pharmazeutischen Präparats gebracht, welches neben dem Wirkstoff für die enterale oder parenterale Applikation geeignete pharmazeutische, organische oder anorganische inerte Trägermaterialien, wie zum Beispiel, Wasser, Gelatine, Gummi arabicum, Milchzucker, Stärke, Magnesiumstearat, Talk, pflanzliche Öle, Polyalkylenglykole usw. enthält.

Die Erfindung betrifft eine Einrichtung (Kit) enthaltend mindestens eine Verbindung der Formel I, II, III, IV oder V.

### Beispiele:

### Beispiel 1

### (2S,4S)-2-Amino-4-(3-fluoropropyl)-pentandisäure

### a) (2S,4S)-4-Allyl-2-tert-butoxycarbonylamino-pentandisäure dimethyl ester

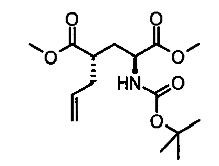

11,01 g (40 mmol) Boc-Glutaminsäure dimethylester (Advanced Chemtech) werden in 160 mL Tetrahydrofuran (THF) gelöst und auf -70°C gekühlt. Innerhalb einer Stunde werden 88 mL (88 mmol) einer 1 M Lösung von Lithium-bis(trimethylsilyl)amid in THF bei dieser Temperatur zugetropft und 2 Stunden bei -70°C nachgerührt. Anschließend werden 14,52 g (120 mmol) Allylbromid zugetropft und nach 2 h bei dieser Temperatur wird das Kältebad entfernt und 200 mL 2 N Salzsäure und 400 mL Essigester zugegeben. Die organische Phase wird abgetrennt, mit Wasser neutral gewaschen, über Natriumsulfat getrocknet, filtriert und das Filtrat eingeengt. Das so erhaltene Rohprodukt wird mit einem Hexan/Essigester-Gradienten an Kieselgel chromatographiert und die entsprechenden Fraktionen vereinigt und eingeengt.
Ausbeute: 3,3 g (26,2 %)

| Elementaranalyse: | | | |
|---|---|---|---|
| ber.: | C 57.13 | H 7.99 | N 4.44 |
| gef: | C 56.97 | H 8.12 | N 4.30 |

### b) (2S,4S)-2-tert-Butoxycarbonylamino-4-(3-hydroxypropyl)-pentandisäure dimethyl ester

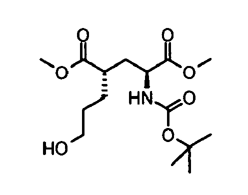

3,15 g (10 mmol) der in Beispiel **1a** beschriebenen Verbindung werden in 50 mL THF gelöst und im Eisbad gekühlt. In ca. 20 Minuten werden im Eis und unter Stickstoff 13,3 mL 1 M Diboran/THF-Komplex in THF zugetropft, 1 h im Eis und über Nacht bei Raumtemperatur gerührt. Anschließend werden 15 mL 1 N Natronlauge und danach 13,3 mL 30%ige wässrige Wasserstoffperoxidlösung zugetropft. Nach 30 Minuten wird mit Wasser verdünnt, THF abdestilliert, die wässrige Restlösung mit Essigester extrahiert. Die organische Phase wird abgetrennt, mit Wasser neutral gewaschen, über Natriumsulfat getrocknet, filtriert und das Filtrat eingeengt. Das so erhaltene Rohprodukt wird mit einem Hexan/Essigester-Gradienten an Kieselgel chromatographiert und die entsprechenden Fraktionen vereinigt und eingeengt.
Ausbeute: 0,6 g (18 %)

| Elementaranalyse: | | | |
|---|---|---|---|
| ber.: | C 54.04 | H 8.16 | N 4.20 |
| gef: | C 53.88 | H 8.25 | N 4.39 |

### c) (2S,4S)-2-tert-Butoxycarbonylamino-4-(3-methanesulfonyloxy-propyl)-pentandisäure dimethyl ester

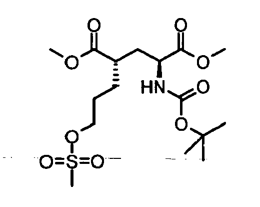

0,17 g (0,5 mmol) der in Beispiel **1b** beschriebenen Hydroxyverbindung werden in Dichlormethan gelöst und im Eisbad gekühlt. Nach Zugabe von 0,30 g (3 mmol) Triethylamin und 115 mg (1 mmol) Methansulfonsäurechlorid wird 2 h im Eis gerührt, eingeengt und das so erhaltene Rohprodukt wird mit einem Hexan/Essigester-Gradienten an Kieselgel chromatographiert und die entsprechenden Fraktionen vereinigt und eingeengt.
Ausbeute: 145 mg (70,5 %)

| Elementaranalyse: | | | | |
|---|---|---|---|---|
| ber.: | C 46.70 | H 7.10 | N 3.40 | S 7.79 |
| gef: | C 46.48 | H 7.22 | N 3.61 | S 7.21 |

### d) (2S,4S)-2-tert-Butoxycarbonylamino-4-(3-fluoropropyl)-pentandisäure dimethyl ester

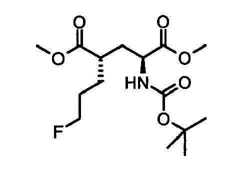

0,33 g (1 mmol) der in Beispiel **1b** beschriebenen Hydroxyverbindung werden in 15 mL Dichlormethan gelöst und im Eis gekühlt. Nach Zugabe von 0,32 g (2 mmol) Diethylaminoschwefeltrifluorid (DAST) wird 1 h im Eis gerührt, mit Wasser gewaschen und die organische Phase über Natriumsulfat getrocknet, filtriert und das Filtrat eingeengt. Das so erhaltene Rohprodukt wird mit einem Dichlormethan/Essigester-Gradienten an Kieselgel chromatographiert und die entsprechenden Fraktionen vereinigt und eingeengt.
Ausbeute: 25 mg (7,5 %)

| Elementaranalyse: | | | | |
|---|---|---|---|---|
| ber.: | C 53.72 | H 7.81 | F 5.66 | N 4.18 |
| gef: | C 53.55 | H 7.94 | F 5.21 | N 4.37 |

### e) (2S,4S)-2-Amino-4-(3-fluoropropyl)-pentandisäure

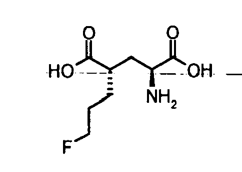

23,5 mg (0,07 mmol) der in Beispiel **1d** beschriebenen Verbindung werden in 2 mL THF gelöst, mit 1 mL 1 N Natronlauge versetzt und 4 h bei Raumtemperatur gerührt. Anschließend wird zur Trockne eingeengt und das erhaltene Rohprodukt in ca. 20 mL 3 N Chlorwasserstoff in Diethylether gelöst, über Nacht gerührt, eingeengt und mehrfach mit Diethylether nachdestilliert. Das so erhaltene Rohprodukt wird mit einem Wasser/Methanol-Gradienten an C18-Kieselgel chromatographiert und die entsprechenden Fraktionen vereinigt und eingeengt.
Ausbeute: 4 mg (27 %)

| Elementaranalyse (berechnet auf die wasserfreie Verbindung): | | | | |
|---|---|---|---|---|
| ber.: | C 46.37 | H 6.81 | F 9.17 | N 6.76 |
| gef: | C 46.11 | H 7.02 | F 8.87 | N 6.93 |

### f) (2S,4S)-2-tert-Butoxycarbonylamino-4-(3-[F-18]fluoropropyl)-pentandisäure dimethyl ester

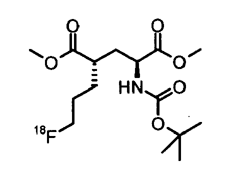

[F-18]Fluorid wurde über die [O-18](p,n)[F-18]-Reaktion in einem Zyklotron hergestellt. Die Isotopenlösung (4 GBq) wurde auf eine Sep-Pack Light QMA-Kartusche gegeben. Das [F-18]Fluorid wurde mit einer Kryptofix 2.2.2/ K₂CO₃-Lösung (5 g K2.2.2, 1 mg K₂CO₃, MeCN (1,5ml), Wasser (0,5ml)) von der Kartusche eluiert. Das Lösungsmittel wurde bei 120 °C in einem Stickstoffstrom unter Zugabe von Acetonitril (dreimal 1 mL) entfernt.
5 mg (12.2 µmol) (2S,4S)-2-tert-Butoxycarbonylamino-4-(3-methanesulfonyloxy-propyl)-pentandisäure dimethyl ester **1c** in 1 mL Acetonitril wurden zugegeben und die resultierende Mischung wurde 10 min bei 110 °C gerührt. Nach Abkühlen auf ca. 60 °C wurde die Mischung durch eine Silica-Plus Kartusche gegeben.
Das Intermediat wurde durch HPLC (C18, Acetonitril/Wasser) gereinigt. Die HPLC-Fraktion wurde mit Wasser (ca. 50 mL) verdünnt und über eine C18-Kartusche gegeben. Das Intermediat wurde mit 1 mL Acetonitril eluiert. In einer Synthesezeit von 80 min wurden 940 MBq (39 % d.c.) (2S,4S)-2-tert-Butoxycarbonylamino-4-(3-[F-18]fluoropropyl)-pentandisäure dimethyl ester **1f** erhalten.

### g) (2S,4S)-2-Amino-4-(3-[F-18]fluoropropyl)-pentandisäure

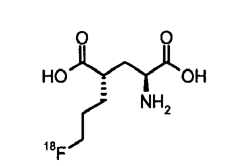

940 MBq 2-tert-Butoxycarbonylamino-4-(3-[F-18]fluoropropyl)-pentandisäuredimethylester **1f** in 1 mL Acetonitril wurden mit 0.5 mL 4N HCl versetzt. Die Mischung wurde für 10 min unter Rühren auf 130 °C (Ölbadtemperatur) erhitzt. Nach Abkühlen auf Raumtemperatur wurde die Lösung durch Zugabe von ca. 650 µL 2N NaOH neutralisiert.
Es wurden 890 MBq (95 % d.c.) (2S,4S)-2-Amino-4-(3-[F-18]fluoropropyl)-pentandisäure **1g** erhalten.

### Beispiel 2

### (2S,4S)-2-Amino-4-(4-fluorobutyl)-pentandisäure

### a) (2S,4S)-2-tert-Butoxycarbonylamino-4-(4-iodobutyl)-pentandisäure dimethyl ester

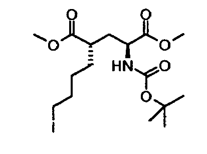

5,51 g (20 mmol) Boc-Glutaminsäure dimethylester werden in 60 mL THF gelöst und auf - 70°C gekühlt. Innerhalb einer Stunde werden 44 mL (44 mmol) einer 1 M Lösung von Lithium-bis(trimethylsilyl)amid in THF bei dieser Temperatur zugetropft und 2 Stunden bei - 70°C nachgerührt. Anschließend werden 18,60 g (60 mmol) 1,4-Diiodbutan zugetropft und nach 2 h bei dieser Temperatur wird das Kältebad entfernt und 100 mL 2 N Salzsäure und 300 mL Essigester zugegeben. Die organische Phase wird abgetrennt, mit Wasser neutral gewaschen, über Natriumsulfat getrocknet, filtriert und das Filtrat eingeengt. Das so erhaltene Rohprodukt wird mit einem Hexan/Essigester-Gradienten an Kieselgel chromatographiert und die entsprechenden Fraktionen vereinigt und eingeengt.
Ausbeute: 1,0 g (11,0%)

| Elementaranalyse: | | | | |
|---|---|---|---|---|
| ber.: | C 42.02 | H 6.17 | I 127.75 | N 3.06 |
| gef: | C 41.78 | H 6.30 | I 27.19 | N 3.22 |

### b) (2S,4S)-2-Amino-4-(4-fluorobutyl)-pentandisäure

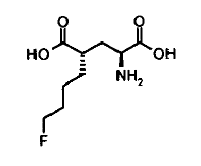

Zu 0.45 g (1 mmol) der in Beispiel **2a** beschriebenen Verbindung in 30 mL Acetonitril wird eine Lösung von 152 mg (1,12 mmol) Silberfluorid in 1,5 mL Wasser zugegeben und über Nacht bei 40°C gerührt. Die erhaltene Suspension wird filtriert, die Lösung zur Trockne eingedampft und das so erhaltene Rohprodukt wird mit einem Hexan/Essigester-Gradienten an Kieselgel chromatographiert und die entsprechenden Fraktionen vereinigt und eingeengt. Der Rückstand wird in 20 mL THF gelöst, mit 10 mL 1 N Natronlauge versetzt und 4 h bei Raumtemperatur gerührt. Anschließend wird zur Trockne eingeengt und das erhaltene Rohprodukt in ca. 70 mL 3 N Chlorwasserstoff in Diethylether gelöst, über Nacht gerührt, eingeengt und mehrfach mit Diethylether nachdestilliert. Die so erhaltene (2S,4S)-2-Amino-4-(4-fluorobutyl)-pentandisäure wird mit einem Wasser/Methanol-Gradienten an C18-Kieselgel chromatographiert und die entsprechenden Fraktionen vereinigt und eingeengt.
Ausbeute: 33 mg (15 %)

| Elementaranalyse (berechnet auf die wasserfreie Verbindung): | | | | |
|---|---|---|---|---|
| ber.: | C 48.86 | H 7.29 | F 8.59 | N 6.33 |
| gef: | C 48.66 | H 7.55 | F 8.20 | N 6.57 |

### c) (2S,4S)-2-tert-Butoxycarbonylamino-4-(4-[F18]fluorobutyl)-pentandisäure dimethyl ester

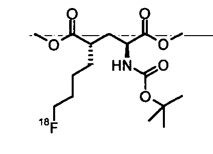

[F-18]Fluorid wurde über die [O-18](p,n)[F-18]-Reaktion in einem Zyklotron hergestellt. Die Isotopenlösung (5.2 GBq) wurde auf eine Sep-Pack Light QMA-Kartusche gegeben. Das [F-18]Fluorid wurde mit einer Kryptofix 2.2.2/ K₂CO₃-Lösung (5 g K2.2.2, 1 mg K₂CO₃, MeCN (1,5ml), Wasser (0,5ml)) von der Kartusche eluiert. Das Lösungsmittel wurde bei 120 °C in einem Stickstoffstrom unter Zugabe von Acetonitril (dreimal 1 mL) entfernt. 5 mg (10.9 µmol) (2S,4S)-2-tert-Butoxycarbonylamino-4-(4-iodobutyl)-pentandisäure dimethyl ester **2a** in 1 mL Acetonitril wurden zugegeben und die resultierende Mischung wurde 10 min bei 110 °C gerührt. Nach Abkühlen auf ca. 60 °C wurde die Mischung durch eine Silica-Plus Kartusche gegeben.
Das Intermediat wurde durch HPLC (C18, Acetonitril/Wasser) gereinigt. Die HPLC-Fraktion wurde mit Wasser (ca. 50 mL) verdünnt und über eine C18-Kartusche gegeben. Das Intermediat wurde mit 1 mL Acetonitril eluiert. In einer Synthesezeit von 85 min. wurden 1,8 GBq (59 % d.c.) (2S,4S)-2-tert-Butoxycarbonylamino-4-(4-[F18]fluorobutyl)-pentandisäure dimethyl ester **2c** erhalten.

### d) (2S,4S)-2-Amino-4-(4-[F-18]fluorobutyl)-pentandisäure

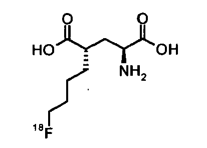

1,8 GBq 2(2S,4S)-2-tert-Butoxycarbonylamino-4-(4-[F18]fluorobutyl)-pentandisäure dimethyl ester **2c** in 1 mL Acetonitril wurden mit 0.5 mL 4N HCl versetzt. Die Mischung wurde für 10 min unter Rühren auf 130 °C (Ölbadtemperatur) erhitzt. Nach Abkühlen auf Raumtemperatur wurde die Lösung durch Zugabe von ca. 700 µL 2N NaOH neutralisiert.
Es wurden 1,7 GBq (94 % d.c.) (2S,4S)-2-Amino-4-(4-[F-18]fluorobutyl)-pentandisäure **2d** hergestellt

### Beispiel 3

### (2S,4S)-2-Amino-4-(2-fluoroethoxy)-pentandisäure

### a) (2S,4S)-4-(2-Bromethoxy)-pyrr olidin-1,2-disäure 1-tert-butyl ester 2-methyl ester

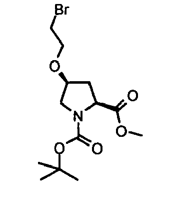

0,98 g (4 mmol) (2S,4S)-4-Hydroxypyrrolidin-1,2-disäure 1-tert-butyl ester 2-methyl ester (ALDRICH) werden in 36 mL 1,2-Dibromethan gelöst und im Eisbad gekühlt. Nach Zugabe von 1,36 g (4 mmol) Tetrabutylammonium-hydrogensulfat werden 18 mL 50%ige wässrige Natronlauge zugegeben, 2 Stunden im Eis und über Nacht bei Raumtemperatur gerührt. Nach Zugabe von 200 mL Wasser und 200 mL Dichlormethan wird die organische Phase noch einmal mit Wasser gewaschen, über Natriumsulfat getrocknet, filtriert und das Filtrat eingeengt. Das so erhaltene Rohprodukt wird mit einem Dichlormethan /Essigester-Gradienten an Kieselgel chromatographiert und die entsprechenden Fraktionen vereinigt und eingeengt.
Ausbeute: 60 mg (4,3 %)

| Elementaranalyse: | | | | |
|---|---|---|---|---|
| ber.: | C 44.33 | H 6.30 | Br 22.69 | N 3.98 |
| gef: | C 44.02 | H 6.33 | Br 22.21 | N 4.11 |

### b) (2S,4S)-4-(2-Bromethoxy)-5-oxo-pyrrolidin-1,2-disäure 1-tert-butyl ester 2-methyl ester

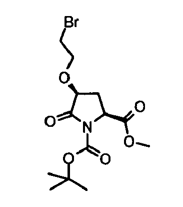

106 mg (0,3 mmol) der in Beispiel 3a beschriebenen Verbindung werden in 10 mL Essigester gelöst. Nach Zugabe von 14 mg (0,06 mmol) Ruthenium(III)-chlorid-hydrat wird eine Lösung von 0,32 g (1,5 mmol) Natriumperiodat in 4 ml Wasser zugegeben, über Nacht gerührt, mit Essigester verdünnt, die organische Phase mit Wasser gewaschen, über Natriumsulfat getrocknet, filtriert und das Filtrat eingeengt. Das so erhaltene Roh¬produkt wird mit einem Hexan/Essigester-Gradienten an Kieselgel chromatographiert und die entsprechenden Fraktionen vereinigt und eingeengt.
Ausbeute: 48 mg (43,7 %)

| Elementaranalyse: | | | | |
|---|---|---|---|---|
| ber.: | C 42.64 | H 5.50 | Br 21.82 | N 3.82 |
| gef: | C 42.33 | H 5.74 | Br 21.17 | N 3.59 |

### c) (2S,4S)-4-(2-Fluorethoxy)-5-oxo-pyrrolidin-1,2-disäure 1-tert-butyl ester 2-methyl ester

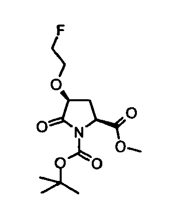

183 mg (0,5 mmol) der in Beispiel **3b** beschriebenen Verbindung in 20 mL Dimethylsulfoxid werden mit 188 mg (0,5 mmol) Kryptofix 222 und 29 mg (0,5 mmol) Kaliumfluorid vesetzt und in der Mikrowelle 30 Minuten bei 100°C umgesetzt. Die Lösung wird im Vakuum eingeengt, der Rückstand zwischen Wasser und Essigester verteilt, die Essigester-Phase mit Wasser gewaschen, über Natriumsulfat getrocknet, filtriert und das Filtrat eingeengt. Das so erhaltene Rohprodukt wird mit einem Hexan/Essigester-Gradienten an Kieselgel chromatographiert und die entsprechenden Fraktionen vereinigt und eingeengt.
Ausbeute: 13,7 mg (9,0 %)

| Elementaranalyse: | | | | |
|---|---|---|---|---|
| ber.: | C 51.14 | H 6.60 | F 6.22 | N 4.59 |
| gef: | C 50.84 | H 6.81 | F 6.00 | N 4.36 |

### d) (2S,4S)-2-Amino-4-(2-fluorethoxy)-pentandisäure

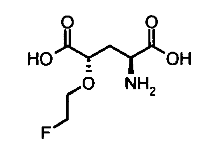

21,4 mg (0,07 mmol) der in Beispiel **3c** beschriebenen Verbindung werden in 2 mL THF gelöst, mit 1 mL 1 N Natronlauge versetzt und 4 h bei Raumtemperatur gerührt. Anschließend wird zur Trockne eingeengt und das erhaltene Rohprodukt in ca. 20 mL 6 N wässriger Salzsäure suspendiert, 6 h bei 80°C gerührt und eingeengt. Das so erhaltene Rohprodukt wird mit einem Wasser/Methanol-Gradienten an C18-Kieselgel chromatographiert und die entsprechenden Fraktionen vereinigt und eingeengt.
Ausbeute: 3,2 mg (22 %)

| Elementaranalyse (berechnet auf die wasserfreie Verbindung): | | | | |
|---|---|---|---|---|
| ber.: | C 40.19 | H 5.78 | F 9.08 | N 6.70 |
| gef: | C 39.82 | H 5.86 | F 8.81 | N 6.96 |

### e) (2S,4S)-4-(2-[F-18]Fluorethoxy)-5-oxo-pyrrolidin-1,2-disäure 1-tert-butyl ester 2-methyl ester

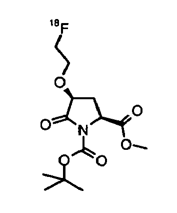

[F-18]Fluorid wurde über die [O-18](p,n)[F-18]-Reaktion in einem Zyklotron hergestellt. Die Isotopenlösung (3,9 GBq) wurde auf eine Sep-Pack Light QMA-Kartusche gegeben. Das [F-18]Fluorid wurde mit einer Kryptofix 2.2.2/ K₂CO₃-Lösung (5 g K2.2.2, 1 mg K₂CO₃, MeCN (1,5ml), Wasser (0,5ml)) von der Kartusche eluiert. Das Lösungsmittel wurde bei 120 °C in einem Stickstoffstrom unter Zugabe von Acetonitril (dreimal 1 mL) entfernt.
5 mg (13.7 µmol) (2S,4S)-4-(2-Bromethoxy)-pyrrolidin-1,2-disäure 1-tert-butyl ester 2-methyl ester **3a** in 1 mL Acetonitril wurden zugegeben und die resultierende Mischung wurde 10 min bei 100 °C gerührt. Nach Abkühlen auf ca. 70 °C wurde die Mischung durch eine Silica-Plus Kartusche gegeben.
Das Intermediat wurde durch HPLC (C18, Acetonitril/Wasser) gereinigt. Die HPLC-Fraktion wurde mit Wasser (ca. 50 mL) verdünnt und über eine C18-Kartusche gegeben. Das Intermediat wurde mit 1 mL Acetonitril eluiert. In einer Synthesezeit von 70 min wurden 1,3 GBq (52 % d.c.) (2S,4S)-4-(2-[F-18]Fluorethoxy)-5-oxo-pyrrolidin-1,2-disäure 1-tert-butyl ester 2-methyl ester **3e** erhalten.

### f) (2S,4S)-2-Amino-4-(2-[F-18]fluorethoxy)-pentandisäure

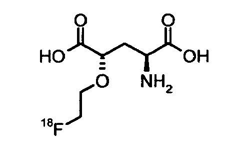

1,3 GBq (2S,4S)-4-(2-[F-18]Fluorethoxy)-5-oxo-pyrrolidin-1,2-disäure 1-tert-butyl ester 2-methyl ester **3e** in 1 mL Acetonitril wurden mit 0.5 mL 4N HCl versetzt. Die Mischung wurde für 10 min unter Rühren auf 130 °C (Ölbadtemperatur) erhitzt. Nach Abkühlen auf Raumtemperatur wurde die Lösung durch Zugabe von ca. 700 µL 2N NaOH neutralisiert.
Es wurden 1,2 GBq (92 % d.c.) (2S,4S)-2-Amino-4-(2-fluorethoxy)-pentandisäure **3f** erhalten.

### Beispiel 4

### Zellexperimente

Die Aufnahme der erfindungsgemäßen Glutaminsäurederivate in Tumorzellen wurde in Zellexperimenten untersucht. Dabei wurde die Aufnahme eines radiomarkierten Glutaminsäurederivates (4R/S-[F-18]F-L-Glutaminsäure) in Gegenwart der erfindungsgemäßen Verbindungen und Kontrollsubstanzen untersucht (Competitionsexperimente). Die erfindungsgemäßen Verbindungen wurden im Überschuss (1 mM) eingesetzt zu 4R/S-[F-18]F-L-Glutaminsäure (Tracer).
Als Kontrolle wurde native L-konfigurierte Glutaminsäure (L-Glu) verwendet, welche in Konzentration von 1 mM L-Glu im Assay eine 87 % ige Inhibierung der Traceraufnahme bedingt.
Überraschenderweise wurde gefunden, dass 4S-konfigurierte Methyl- und Hydroxy-Derivate eine deutlich bessere Inhibierung der Tracer-Aufnahme zeigen, als die entsprechenden 4R-konfigurierten Derivate.
4S-(3-Fluoropropyl)-L-Glu zeigte dabei eine deutlich bessere Inhibition als andere untersuchte Derivate. So konnte durch 1 mM 4S-(3-Fluoropropyl)-L-Glu die Tracer-Aufnahme zu >94 % auf 5.4 % reduziert werden.

**Tabelle 1: Untersuchung der biologischen Aktivität von erfindungsgemäßen Verbindungen im Kompetitionszellexperiment. (A549 Zellen, 10 min Inkubation mit 0.250 MBq 4R/S-[F-18]F-L-Glu in PBS-Puffer, Kompetitorkonzentration 1 mM)**

| | **% Tracer-Aufnahme** | S.D. |
|---|---|---|
| **Kontrolle** | 100.0 | 3.5 |
| **L-Glu** | 12.6 | 1.6 |
| **4-Fluoro-D/L-Glu** | 16.3 | 2.5 |
| **(4S)-Hydroxy-L-Glu** | 13.2 | 1.8 |
| **(4S)-Methyl-L-Glu** | 7.7 | 1.9 |
| **4S-(3-Fluoropropyl)-L-Glu** | 5.4 | 2.2 |
| **(4R)-Hydroxy-L-Glu** | 29.6 | 4.2 |
| **(4R)-Methyl-L-Glu** | 33.5 | 5.5 |

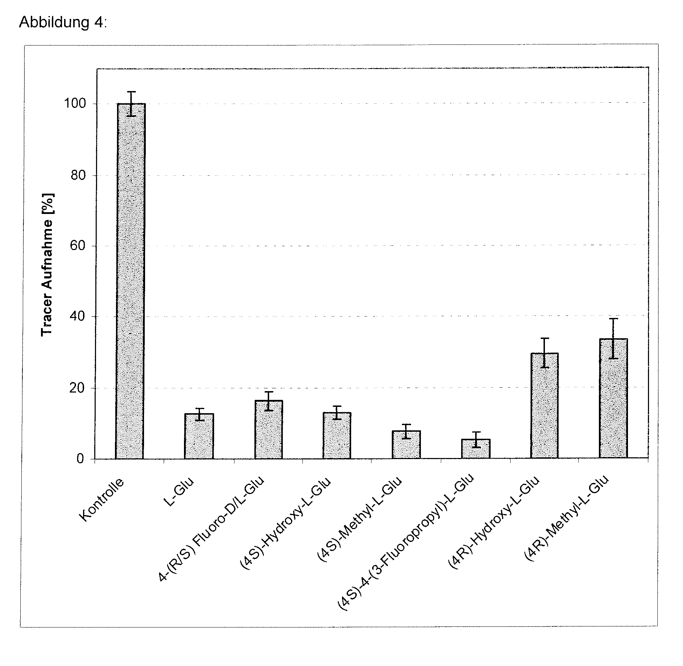

Abbildung 4: Graphische Darstellung der Inhibition von 4R/S-[F-18]F-L-Glu-Zellaufnahme durch R- und S-konfigurierte Glutaminsäurederivaten in A549-Zellen (humanes nichtkleinzelliges Bronchialkarzinom) nach 10 min. Inkubation.

### Zellaufnahme von S-konfigurierten, F-18-markierten Glutaminsäurederivaten

Nach F-18-Markierung wurde (2S,4S)-2-Amino-4-(3-[F-18]fluoropropyl)-pentandisäure in Zellexperimenten an A549 und H460-Tumorzellen (beides humane nicht-kleinzeilige Bronchialkarzinom-Zelllinien) untersucht. Dabei wurde eine zeitliche Abhängigkeit der Zellaufnahme beobachtet. Nach 30 min. Inkubation konnte eine Aufnahme von 899000 cpm pro 100.000 Zellen für (2S,4S)-2-Amino-4-(3-[F-18]fluoropropyl)-pentandisäure gemessen werden. Damit reichert sich (2S,4S)-2-Amino-4-(3-[F-18]fluoropropyl)-pentandisäure stärker in diesen Tumorzellen an, als der "Goldstandard" [F-18]FDG nach 30 min. Inkubation.

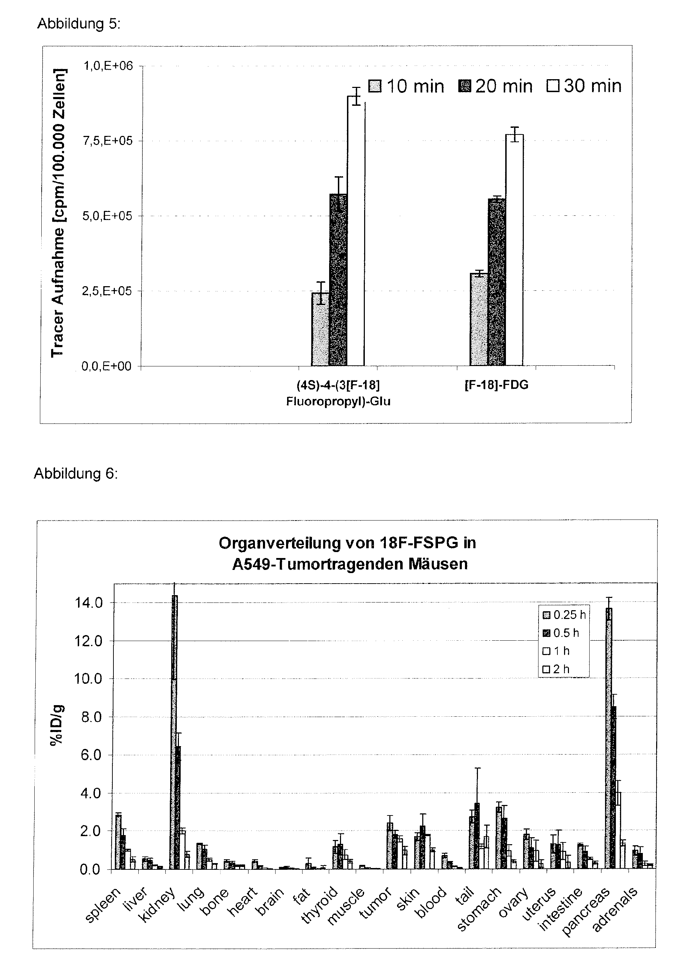

Abbildung 5. Zeitabhängige Zellaufnahme von (2S,4S)-2-Amino-4-(3-[F-18]fluoropropyl)-pentandisäure im Vergleich mit [F-18]FDG. Für alle F-18 markierten Verbindungen wurde eine zeitliche Abhängigkeit der intrazellulären Radioaktivität beobachtet. Nach 30 min wurden 840000 cpm /100000 Zellen von (2S,4S)-2-Amino-4-(3-[F-18]fluoropropyl)-pentandisäure aufgenommen. Von [F-18]FDG wurden 770000 cpm/100000 Zellen nach 30 min aufgenommen.

### Tierexperimente

(2S,4S)-2-Amino-4-(3-[F-18]fluoropropyl)-pentandisäure (15 MBq) wurde in einem Organverteilungsexperiment in A549-Tumortragenden Mäusen untersucht.

Nach 0,25 h wurde 2,4 % Injizierte Dosis (ID)/ g im Tumor gemessen, nach 1 h liegt die Aufnahme im Tumor bei 1,6 % ID/ g. Eine transiente Aufnahme bzw. Exkretionen wurde in den Nieren bzw. Pankreas beobachtet. So wurde in diesen Organen nach 0,25 h eine Aufnahme von 14,4 %ID/ g bzw. 13,6%ID/ g beobachtet. Nach 1 h reduzierte sich die Aktivität in diesen auf 2 % ID/ g bzw. 4 % ID/ g.
Die Knochenaufnahme lag bei allen Zeitpunkten <0,5%ID/g.

**Tabelle 2: Organverteilung nach i.v. Applikation von (2S,4S)-2-Amino-4-(3-[F-18]fluoropropyl)-pentandisäure (18FSPG) in A549-Tumortragende Mäuse.**

| **Zeitpunkt** | **0,25 h** | | **0,5 h** | | **1,0 h** | | **2,00 h** | |
|---|---|---|---|---|---|---|---|---|
| **%Dosis/g** | | S.D. | | S.D. | | S.D. | | S.D. |
| **Milz / spleen** | **2.87** | 0.11 | **1.76** | 0.38 | **1.02** | 0.07 | **0.53** | 0.13 |
| **Leber / liver** | **0.55** | 0.11 | **0.46** | 0.12 | **0.21** | 0.03 | **0.11** | 0.03 |
| **Niere / kidney** | **14.38** | 4.41 | **6.44** | 0.71 | **2.01** | 0.17 | **0.80** | 0.17 |
| **Lunge / lung** | **1.35** | 0.03 | **1.08** | 0.19 | **0.50** | 0.08 | **0.29** | 0.02 |
| **Knochen / bone** | **0.44** | 0.06 | **0.37** | 0.07 | **0.20** | 0.03 | **0.20** | 0.03 |
| **Herz / heart** | **0.42** | 0.08 | **0.20** | 0.02 | **0.08** | 0.01 | **0.05** | 0.00 |
| **Hirn / brain** | **0.09** | 0.01 | **0.10** | 0.05 | **0.06** | 0.01 | **0.05** | 0.01 |
| **Fett / fat** | **0.31** | 0.30 | **0.08** | 0.03 | **0.04** | 0.01 | **0.09** | 0.11 |
| **Schilddrüse / thyroid** | **1.17** | 0.33 | **1.31** | 0.55 | **0.77** | 0.24 | **0.42** | 0.10 |
| **Muskel / muscle** | **0.17** | 0.03 | **0.09** | 0.00 | **0.05** | 0.00 | **0.03** | 0.00 |
| ***Tumor* / *tumor*** | ***2.42*** | *0.39* | ***1.82*** | *0.21* | ***1.58*** | *0.15* | ***0.97*** | *0. 21* |
| **Haut / skin** | **1.71** | 0.20 | **2.26** | 0.64 | **1.80** | 0.04 | **1.01** | 0.10 |
| **Blut / blood** | **0.72** | 0.12 | **0.35** | 0.06 | **0.13** | 0.01 | **0.07** | 0.01 |
| **Schwanz / tail** | **2.74** | 0.34 | **3.45** | 1.86 | **1.19** | 0.13 | **1.69** | 0.60 |
| **Magen / stomach** | **3.25** | 0.27 | **2.64** | 0.70 | **0.96** | 0.29 | **0.39** | 0.09 |
| **Ovar / ovary** | **1.82** | 0.29 | **1.13** | 0.48 | **0.94** | 0.56 | **0.28** | 0.20 |
| **Uterus / uterus** | **1.30** | 0.47 | **1.27** | 0.74 | **0.93** | 0.44 | **0.37** | 0.33 |
| **Darm / intestine** | **1.27** | 0.06 | **0.90** | 0.28 | **0.52** | 0.06 | **0.32** | 0.07 |
| **Bauchspdr. / pankreas** | **13.64** | 0.59 | **8.51** | 0.65 | **3.99** | 0.66 | **1.35** | 0.15 |
| **Nebenniere** / **adrenals** | **0.94** | 0.23 | **0.78** | 0.38 | **0.27** | 0.11 | **0.20** | 0.04 |

### PET/CT-Bildgebung mit (2S,4S)-2-Amino-4-(3-[F-18]fluoropropyl)-pentandisäure in A549-Tumortragenden Mäusen

60 min nach i.v. Applikation von 10 MBq (2S,4S)-2-Amino-4-(3-[F-18]fluoropropyl)-pentandisäure in A549-Tumortragende Mäuse wurde die Datenakquisition mit einem PET/CT-Scanner (Inveon) für 20 min gestartet. Die Bildanalyse zeigt eine hohe Aufnahme von (2S,4S)-2-Amino-4-(3-[F-18]fluoropropyl)-pentandisäure den A549-Tumor.

### PET mit (4S)-4-(3-[F-18]Fluoropropyl)-L-Glu in H460-Tumortragenden Ratten

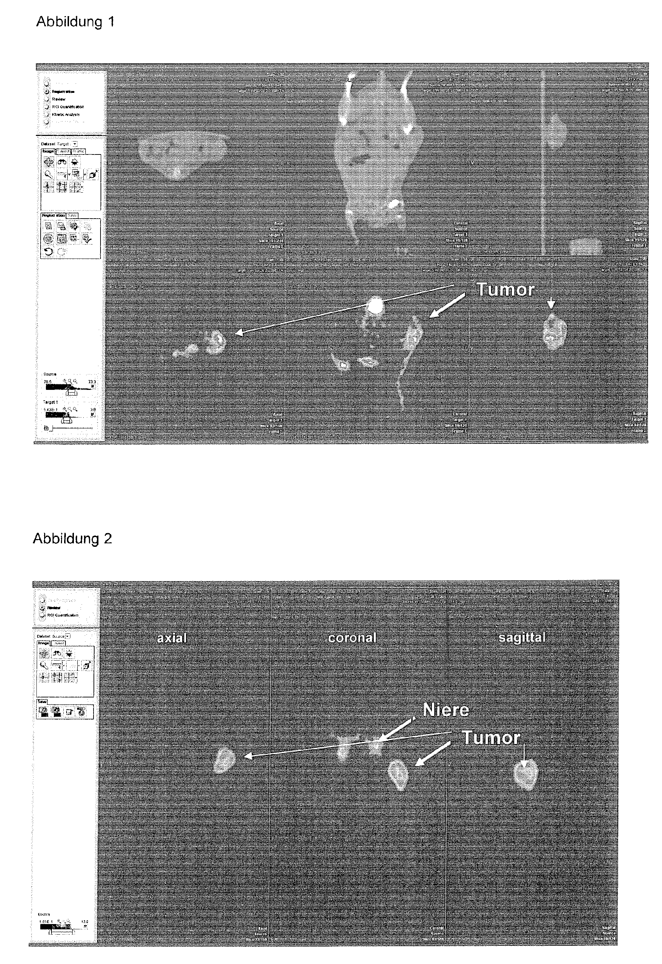

80 min nach i.v. Applikation von 18 MBq (2S,4S)-2-Amino-4-(3-[F-18]fluoropropyl)-pentandisäure in H460-tumortragenden Ratten wurde die Datenakquisition mit einem PET-Scanner (Inveon) für 20 min gestartet. Die Bildanalyse zeigt eine hohe Aufnahme von (2S,4S)-2-Amino-4-(3-[F-18]fluoropropyl)-pentandisäure in den H460-Tumor (ca. 3%ID/g). Abbildung 1.

Abbildung 2. PET mit (2S,4S)-2-Amino-4-(3-[F-18]fluoropropyl)-pentandisäure in H460-Tumortragenden Ratten (Schnittbild-Analyse, 80-100 min nach i.v. Applikation von 18 MBq (4S)-4-(3-[F-18]Fluoropropyl)-L-Glu)

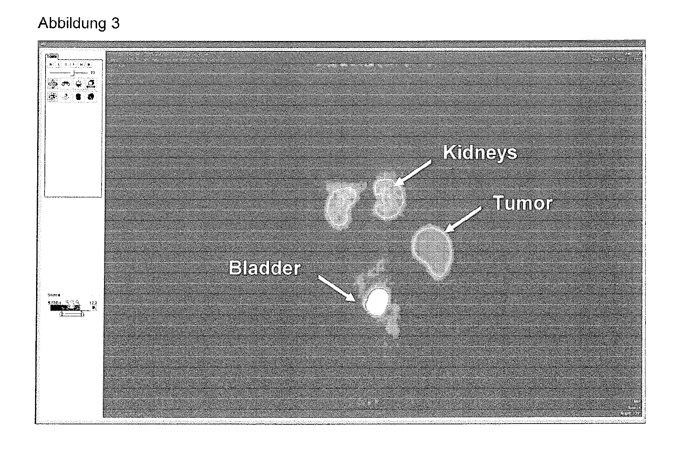

Abbildung 3. PET mit (2S,4S)-2-Amino-4-(3-[F-18]fluoropropyl)-pentandisäure in H460-Tumortragenden Ratten (Maximum-Intensity-Projection, 80-100 min nach i.v. Applikation von 18 MBq (2S,4S)-2-Amino-4-(3-[F-18]fluoropropyl)-pentandisäure)

## Patentansprüche

**1.** Verbindungen der allgemeinen Formel I 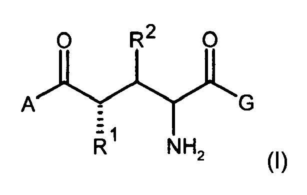 worin
A für
a) Hydroxyl,
b) verzweigtes oder unverzweigtes C₁-C₅ alkoxy,
c) verzweigtes oder unverzweigtes Hydroxy C₁-C₅ Alkoxy,
d) verzweigtes oder unverzweigtes O-C₁-C₅ Alkyl-(O-C₁-C₄ alkyl)ₙ-O-C₁-C₄ alkyl,
e) N(C₁-C₅ Alkyl)₂,
f) NH₂,
g) N(H)-L,
h) O-L oder
i) O-Z
steht,
G für
a) Hydroxyl,
b) O-Z,
b) verzweigtes oder unverzweigtes O-C₁-C₅ Alkyl,
c) verzweigtes oder unverzweigtes O-C₂-C₅ Alkenyl,
d) verzweigtes oder unverzweigtes O-C₁-C₅ Alkyl-(O-C₁-C₄ alkyl)ₙ-O-C₁-C₄ alkyl,
e) verzweigtes oder unverzweigtes O-C₂-C₅ Alkinyl, oder
f) Triphenylmethoxy
steht,
R¹ und R² für
i) Wasserstoff,
j) verzweigtes oder unverzweigtes ¹⁸F-C₂-C₅ Alkoxy,
k) verzweigtes oder unverzweigtes ¹⁸F-C₁-C₅ Alkyl,
l) verzweigtes oder unverzweigtes ¹⁸F-C₂-C₅ Alkenyl,
m) verzweigtes oder unverzweigtes ¹⁸F-C₂-C₅ Alkinyl,
n) Hydroxyl,
o) verzweigtes oder unverzweigtes C₁-C₅ Alkyl oder
p) verzweigtes oder unverzweigtes C₁-C₅ Alkoxy
steht,
mit der Maßgabe, dass einer der Substituenten R¹ oder R² genau ein ¹⁸F Isotop beinhaltet und der jeweils andere Substituent kein ¹⁸F Isotop beinhaltet, mit der Maßgabe dass R¹ nicht Wasserstoff ist,
L für
a) verzweigtes oder unverzweigtes C₁-C₅ Alkyl,
b) verzweigtes oder unverzweigtes C₂-C₅ Alkenyl,
c) verzweigtes oder unverzweigtes C₁-C₅ Alkyl-(O-C₁-C₄ alkyl)ₙ-O-C₁-C₄ alkyl oder
d) verzweigtes oder unverzweigtes C₂-C₅ Alkinyl,
steht,
Z für ein Metallkationenequivalent steht, und
wobei
n = 0, 1, 2 oder 3 ist.

**2.** Verbindungen nach Anspruch 1, **dadurch gekennzeichnet, dass** A für Hydroxyl, verzweigtes oder unverzweigtes C₁-C₅ Alkoxy, oder NH₂ steht.

**3.** Verbindung nach Ansprüchen 1 oder 2, **dadurch gekennzeichnet, dass** A für NH₂ steht.

**4.** Verbindungen nach Ansprüchen 1 bis 3, **dadurch gekennzeichnet, dass** R¹ ausgewählt ist aus der Gruppe ¹⁸F-methoxy, ¹⁸F-ethoxy, ¹⁸F-propoxy, ¹⁸F-methyl, ¹⁸F-ethyl und ¹⁸F-propyl und R² Wasserstoff ist.

**5.** Verbindung nach Ansprüchen 1 bis 3, **dadurch gekennzeichnet, dass** R¹ für ¹⁸F und R² für Wasserstoff steht.

**6.** Verbindung nach Ansprüchen 1 bis 5, **dadurch gekennzeichnet, dass** G ausgewählt ist aus der Gruppe Hydroxyl, verzweigtes oder unverzweigtes O-C₁-C₄ Alkyl.

**7.** Verbindung nach Anspruch 6, **dadurch gekennzeichnet, dass** G Methoxy ist.

**8.** Verbindung nach Ansprüchen 1 bis 7, **dadurch gekennzeichnet, dass** Z ausgewählt ist aus der Gruppe Mg²⁺, Ca²⁺, Na⁺ und K⁺.

**9.** Verbindung nach Anspruch 1 ausgewählt aus der Gruppe von Verbindungen mit der Formel:

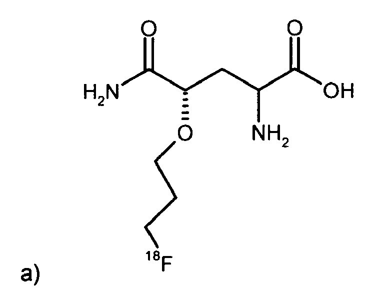

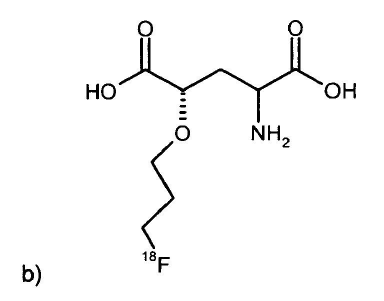

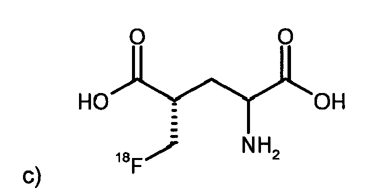

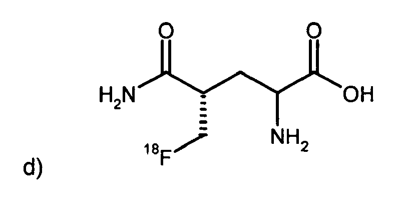

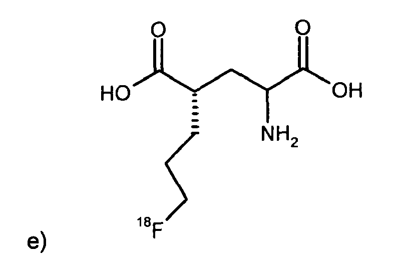

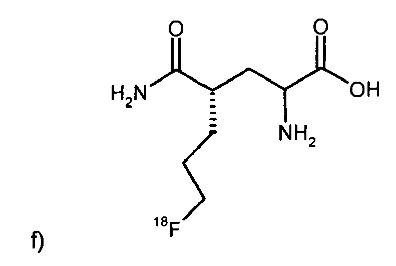

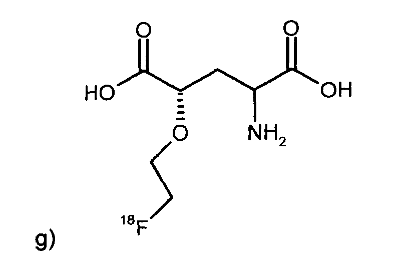

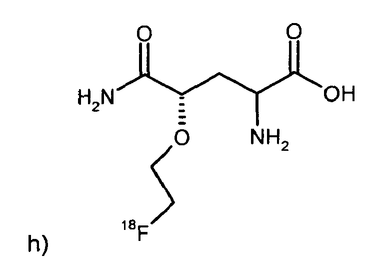

**10.** Verbindungen der allgemeinen Formel (II): 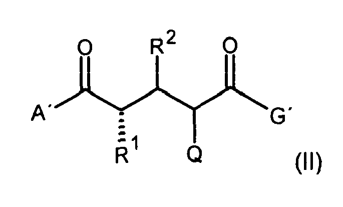 worin,
A' für
a) Hydroxyl,
b) verzweigtes oder unverzweigtes C₁-C₅ alkoxy,
c) verzweigtes oder unverzweigtes Hydroxy C₁-C₅ Alkoxy,
d) verzweigtes oder unverzweigtes O-C₁-C₅ Alkyl-(O-C₁-C₄ alkyl)ₙ-O-C₁-C₄ alkyl,
e) N(C₁-C₅ Alkyl)₂,
f) NH₂,
g) N(H)-L', oder
h) O-L'
steht,
G' für
a) hydroxyl,
b) O-Z',
c) verzweigtes oder unverzweigtes O-C₁-C₅ Alkyl,
d) verzweigtes oder unverzweigtes O-C₂-C₅ Alkenyl,
e) verzweigtes oder unverzweigtes O-C₁-C₅ Alkyl-(O-C₁-C₄ alkyl)ₙ-O-C₁-C₄ alkyl,
f) verzweigtes oder unverzweigtes O-C₂-C₅ Alkinyl, oder
g) Triphenylmethoxy
steht,
R¹ und R² für
a) Wasserstoff,
i) verzweigtes oder unverzweigtes ¹⁸F C₂-C₅ Alkoxy,
j) verzweigtes oder unverzweigtes ¹⁸F-C₁-C₅ Alkyl,
k) verzweigtes oder unverzweigtes ¹⁸F-C₂-C₅ Alkenyl,
l) verzweigtes oder unverzweigtes ¹⁸F-C₂-C₅ Alkinyl,
m) Hydroxyl,
n) verzweigtes oder unverzweigtes C₁-C₅ Alkyl, oder
o) verzweigtes oder unverzweigtes C₁-C₅ Alkoxy
steht,
mit der Maßgabe, dass genau einer der Substituenten R¹ oder R² genau ein ¹⁸F Isotop und der jeweils andere Substituent kein ¹⁸F Isotop beinhaltet, mit der Maßgabe dass R¹ nicht Wasserstoff ist,
Q für
a) N(H)-tert-Butoxycarbonyl,
b) N(H)-Allyloxycarbonyl,
c) N(H)-Benzyloxycarbonyl,
d) N(H)-Ethoxycarbonyl,
e) N(H)-Methoxycarbonyl,
f) N(H)-Propoxycarbonyl,
e) N(H)-2,2,2-Trichlorethoxycarbonyl,
f) N(H)-1,1-Dimethylpropinyl,
g) N(H)-1-Methyl-1-phenyl-ethoxycarbonyl,
h) N(H)-1-Methyl-1-(4-biphenylyl)-ethoxycarbonyl,
i) N(H)-cyclobutylcarbonyl,
j) N(H)-1-Methylcyclobutylcarbonyl,
k) N(H)-Vinylcarbonyl,
l) N(H)-Allylcarbonyl,
m) N(H)-Adamantylcarbonyl,
n) N(H)-Diphenylmethylcarbonyl,
o) N(H)-Cinnamylcarbonyl,
p) N(H)-Formyl,
q) N(H)-Benzoyl,
r) N(H)-Trityl,
s) N(H)-p-Methoxyphenyl-diphenylmethyl,
t) N(H)-di-(p-Methoxyphenyl)-phenylmethyl,
u) 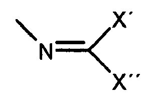 oder
v) N-(tert-Butoxycarbonyl)₂,
steht,
L' für
a) verzweigtes oder unverzweigtes C₁-C₅ Alkyl,
b) verzweigtes oder unverzweigtes C₂-C₅ Alkenyl,
c) verzweigtes oder unverzweigtes C₁-C₅ Alkyl-(O-C₁-C₄ alkyl)ₙ-O-C₁-C₄ alkyl, oder
d) verzweigtes oder unverzweigtes C₂-C₅ Alkinyl
steht,
X' und X'' unabhängig voneinander für
a) verzweigtes oder unverzweigtes C₁-C₅ Alkyl,
b) substituiertes oder unsubstituiertes Aryl,
c) substituiertes oder unsubstituiertes Aralkyl oder
d) substituiertes oder unsubstituiertes Heteroaryl
steht,
Z' für ein Metallkationenequivalent
steht, und
wobei n = 0, 1, 2 oder 3 ist.

**11.** Verbindungen nach Anspruch 10, **dadurch gekennzeichnet, dass** A' für Hydroxyl, verzweigtes oder unverzweigtes C₁-C₅ Alkoxy oder NH₂ steht.

**12.** Verbindungen nach Anspruch 11, **dadurch gekennzeichnet, dass** A' für Methoxy steht.

**13.** Verbindungen nach Ansprüchen 10 bis 12, **dadurch gekennzeichnet, dass** R¹ ausgewählt ist aus der Gruppe ¹⁸F-ethoxy, ¹⁸F-propoxy, ¹⁸F-methyl, ¹⁸F-ethyl und ¹⁸F-propyl und R² Wasserstoff ist.

**14.** Verbindung nach Ansprüchen 10 bis 13, **dadurch gekennzeichnet**, G ausgewählt ist aus der Gruppe Hydroxyl, und verzweigtes oder unverzweigtes O-C₁-C₄ Alkyl.

**15.** Verbindung nach Anspruch 14, **dadurch gekennzeichnet, dass** G Methoxy ist.

**16.** Verbindung nach Ansprüchen 10 bis 15, **dadurch gekennzeichnet, dass** Z' ausgewählt ist aus der Gruppe Na⁺, K⁺, Ca²⁺ und Mg²⁺.

**17.** Verbindung nach Ansprüchen 10 bis 16, **dadurch gekennzeichnet, dass** Q ausgewählt ist aus der Gruppe N(H)-tert-Butoxycarbonyl, N(H)-Benzyloxycarbonyl und 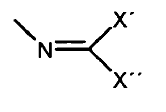 worin
X und X' unabhängig voneinander für
a) verzweigtes oder unverzweigtes C₁-C₅ Alkyl,
b) substituiertes oder unsubstituiertes Aryl,
c) substituiertes oder unsubstituiertes Aralkyl oder
d) substituiertes oder unsubstituiertes Heteroaryl
steht..

**18.** Verbindung nach Anspruch 17 **dadurch gekennzeichnet, dass** Q für N(H)-tert-Butoxycarbonyl steht.

**19.** Verfahren zur Herstellung von Verbindungen der allgemeinen Formel (I) gemäß Ansprüchen 1 bis 9
indem
- eine oder mehrere vorhandene Schutzgruppen einer Verbindung nach Formel (II) gemäß Ansprüchen 10 bis 18 abgespalten wird oder werden.

**20.** Verfahren zur Herstellung von Verbindungen der allgemeinen Formel (II) gemäß Ansprüchen 10 bis 18
indem
- eine Verbindung nach Formel (III) gemäß Anspruch 24 mit F-18 Fluorid umgesetzt wird.

**21.** Verbindungen gemäß den Ansprüchen 1 bis 18 zur Verwendung als Arzneimittel.

**22.** Verbindungen gemäß den Ansprüchen 1 bis 18 zur Verwendung für Bildgebung bei Tumorerkrankungen.

**23.** Verwendung von Verbindungen gemäß den Ansprüchen 1 bis 18 zur Herstellung eines Arzneimittels für Bildgebung bei Tumorerkrankungen.

**24.** Verbindungen der Formel (III) 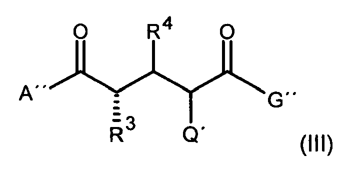 worin
A" für
a) verzweigtes oder unverzweigtes C₁-C₅ alkoxy,
b) verzweigtes oder unverzweigtes Hydroxy C₁-C₅ Alkoxy,
c) verzweigtes oder unverzweigtes O-C₁-C₅ Alkyl-(O-C₁-C₄ alkyl)ₙ-O-C₁-C₄ alkyl,
d) N(C₁-C₅ Alkyl)₂,
e) NH₂,
f) N(H)-U'
g) N(H)-L" oder
h) O-L''
steht,
G" für
a) O-Z'',
b) verzweigtes oder unverzweigtes O-C₁-C₅ Alkyl,
c) verzweigtes oder unverzweigtes O-C₂-C₅ Alkenyl,
d) verzweigtes oder unverzweigtes O-C₁-C₅ Alkyl-(O-C₁-C₄ alkyl)ₙ-O-C₁-C₄ alkyl,
e) verzweigtes oder unverzweigtes O-C₂-C₅ Alkinyl, oder
f) Triphenylmethoxy
steht,
R³ und R⁴ für
a) Wasserstoff,
b) verzweigtes oder unverzweigtes E-C₂-C₅ Alkoxy,
c) verzweigtes oder unverzweigtes E-C₁-C₅ Alkyl,
d) verzweigtes oder unverzweigtes E-C₂-C₅ Alkenyl,
e) verzweigtes oder unverzweigtes E-C₂-C₅ Alkinyl,
f) Hydroxyl,
g) verzweigtes oder unverzweigtes C₁-C₅ Alkyl, oder
h) verzweigtes oder unverzweigtes C₁-C₅ Alkoxy
steht, mit der Maßgabe, dass genau einer der Substituenten R³ oder R⁴ ein E beinhaltet und der jeweils andere Substituent kein E beinhaltet, mit der Maßgabe dass R³ nicht Wasserstoff ist,
E für
a) Chloro,
b) Bromo,
c) Methansulfonyloxy,
d) Trifluormethansulfonyloxy,
e) Nonafluorbutyloxy,
f) Tosyloxy oder
g) Iodo
steht,
Q' für
a) N(H)-tert-Butoxycarbonyl,
b) N(H)-Allyloxycarbonyl,
c) N(H)-Benzyloxycarbonyl,
d) N(H)-Ethoxycarbonyl,
e) N(H)-Methoxycarbonyl,
f) N(H)-Propoxycarbonyl,
g) N(H)-2,2,2-Trichlorethoxycarbonyl,
h) N(H)-1,1-Dimethylpropinyl,
i) N(H)-1-Methyl-1-phenyl-ethoxycarbonyl,
j) N(H)-1-Methyl-1-(4-biphenylyl)-ethoxycarbonyl,
k) N(H)-Cyclobutylcarbonyl,
l) N(H)-1-Methylcyclobutylcarbonyl,
m) N(H)-Vinylcarbonyl,
n) N(H)-Allylcarbonyl,
o) N(H)-Adamantylcarbonyl,
p) N(H)-Diphenylmethylcarbonyl,
q) N(H)-Cinnamylcarbonyl,
r) N(H)-Formyl,
s) N(H)-Benzoyl,
t) N(H)-Trityl,
u) N(H)-p-Methoxyphenyl-diphenylmethyl,
v) N(H)-di-(p-Methoxyphenyl)-phenylmethyl,
w) 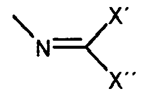 oder
x) N-(tert-Butoxycarbonyl)₂,
steht,
L" für
a) verzweigtes oder unverzweigtes C₁-C₅ Alkyl,
b) verzweigtes oder unverzweigtes C₂-C₅ Alkenyl,
c) verzweigtes oder unverzweigtes C₁-C₅ Alkyl-(O-C₁-C₄ alkyl)ₙ-O-C₁-C₄ alkyl oder
d) verzweigtes oder unverzweigtes C₂-C₅ Alkinyl
steht,
X' and X'' unabhängig voneinander für
a) verzweigtes oder unverzweigtes C₁-C₅ Alkyl,
b) substituiertes oder unsubstituiertes Aryl,
c) substituiertes oder unsubstituiertes Alkylaryl oder
d) substituiertes oder unsubstituiertes Heteroaryl
steht,
Z" für ein Metallkationenequivalent steht, und
wobei n = 0, 1, 2 oder 3 ist.

**25.** Verwendung von Verbindungen der Formel (IV) zur Herstellung von Verbindungen der Formel (I) oder (II): 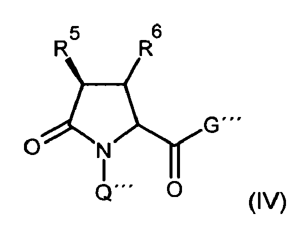 worin
G''' für
a) verzweigtes oder unverzweigtes O-C₁-C₅ Alkyl,
b) verzweigtes oder unverzweigtes O-C₂-C₅ Alkenyl,
c) verzweigtes oder unverzweigtes O-C₁-C₅ Alkyl-(O-C₁-C₄ alkylkO-C₁-C₄ alkyl,
d) verzweigtes oder unverzweigtes O-C₂-C₅ Alkinyl, oder
e) Triphenylmethoxy
steht,
R⁵ und R⁶ für
a) Wasserstoff
b) Hydroxyl,
c) verzweigtes oder unverzweigtes C₁-C₅ Alkyl,
d) verzweigtes oder unverzweigtes C₁-C₅ Alkoxy oder
e) R⁷-E'
steht, mit der Maßgabe, dass genau einer der Substituenten R⁵ oder R⁶ ein E' beinhaltet und der jeweils andere Substituent kein E' beinhaltet, mit der Maßgabe
dass R⁵ nicht Wasserstoff ist,
E' für
a) Chloro,
b) Bromo,
c) Methansulfonyloxy,
d) Trifluormethansulfonyloxy,
e) Nonafluorbutyloxy,
f) Tosyloxy oder
g) Iodo
steht,
R⁷ für
a) verzweigtes oder unverzweigtes C₂-C₅ Alkoxy,
b) verzweigtes oder unverzweigtes C₁-C₅ Alkyl,
c) verzweigtes oder unverzweigtes C₂-C₅ Alkenyl, oder
d) verzweigtes oder unverzweigtes C₂-C₅ Alkinyl
steht,
Q"' für
a) N-tert-Butoxycarbonyl,
b) N-Allyloxycarbonyl,
c) N-Benzyloxycarbonyl,
d) N-Ethoxycarbonyl,
e) N-Methoxycarbonyl,
f) N-Propoxycarbonyl,
g) N-2,2,2-Trichlorethoxycarbonyl,
h) Wasserstoff,
i) N-1-Methyl-1-phenyl-ethoxycarbonyl,
j) N-1-Methyl-1-(4-biphenylyl)-ethoxycarbonyl,
k) N-cyclobutylcarbonyl,
l) N-1-Methylcyclobutylcarbonyl,
m) N-Vinylcarbonyl,
n) N-Allylcarbonyl,
o) N-Adamantylcarbonyl,
p) N-Diphenylmethylcarbonyl,
q) N-Cinnamylcarbonyl,
r) N-Formyl, oder
s) N-Benzoyl,
t) N(H)-Trityl,
u) N(H)-p-Methoxyphenyl-diphenylmethyl,
v) N(H)-di-(p-Methoxyphenyl)-phenylmethyl,
w) 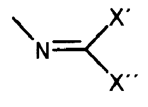 oder
x) N-(tert-Butoxycarbonyl)₂,
steht,
X' and X'' unabhängig voneinander für
a) verzweigtes oder unverzweigtes C₁-C₅ Alkyl,
b) substituiertes oder unsubstituiertes Aryl,
c) substituiertes oder unsubstituiertes Alkylaryl oder
d) substituiertes oder unsubstituiertes Heteroaryl
steht, und
wobei n = 0, 1, 2 oder 3 ist.

**28.** Bildgebung-Kit enthaltend Verbindungen der allgemeinen Formel III oder IV.

**29.** Pharmazeutische Zusammensetzung enthaltend Verbindungen der allgemeinen Formel I, II, III oder IV und geeignete pharmazeutische Träger Substanzen.

**30.** Verbindungen nach Ansprüchen 1 bis 9, 10 bis18, und Verbindungen der allgemeinen Formel IV **dadurch gekennzeichnet dass** die Verbindungen für Bildgebung in einem Dosisbereich von 37 - 600 MBq geeignet sind.

**31.** Verbindungen nach Anspruch 30 **dadurch gekennzeichnet dass** die Verbindungen in einem Dosisbereich von 150 MBq - 370 MBq besonders geeignet sind.
